# EUROPEAN PATENT APPLICATION

(11) **EP 1 793 003 A1**
(43) Date of publication of application: **06.06.2007**
(21) Application number: 05026117.1
(22) Date of filing: 30.11.2005
(51) Int. Cl.: C12Q 1/68

(54) **CITED4 as prognostic marker in oligodendroglial tumors**

(71) Applicant: Deutsches Krebsforschungszentrum, Stiftung des öffentlichen Rechts, 69120 Heidelberg (DE)
(72) Inventor: Tews, Björn, 69221 Dossenheim (DE); Hahn, Meinhard, Dr., 35390 Giessen (DE); Lichter, Peter, Prof. Dr., 69251 Gailberg (DE); Reifenberger, Guido, Prof. Dr., 40225 Düsseldorf (DE); Roerig, Peter, Dr., 47057 Duisburg (DE); Felsberg, Jörg, Dr., 41541 Dormagen (DE); von Deimling, Andreas, Prof. Dr., 13467 Berlin (DE); Hartmann, Christian, Dr., 13353 Berlin (DE)
(74) Representative: Graf von Stosch, Andreas

(57) **Abstract**

The present invention provides novel (diagnostic) methods of detecting cancer of the central nervous system, preferably oligodendrogliomas, by detecting the presence of CITED4-nucleic acid sequences in a biological sample. The present invention furthermore provides a (diagnostic) method for determining the degree of methylation of the promoter region of the CITED4 gene in a biological sample. As a further embodiment, a method for gene-therapy suitable for treating or preventing cancers of the central nervous system, preferably oligodendrogliomas, is provided. Finally, nucleic acid sequences, amino acid sequences, vectors, pharmaceutical compositions and kits, suitable in these inventive methods are disclosed herewith.

SEQUENCE LISTING

<110>  DKFZ Deutsches Krebsforschungszentrum

<120>  CITED4 as prognostic marker in oligodendroglial tumors

<130>  DK01P001EP

<160>  42

<170>  PatentIn version 3.3

<210>  1
<211>  552
<212>  DNA
<213>  Homo sapiens

<220>
<221>  misc_feature
<223>  Description of sequence:  (cDNA) encoding sequence of CITED4

<220>
<221>  CDS
<222>  (1)..(552)

<400>  1
atg gcc gac cac ctg atg ctc gcc gag ggc tac cgc ctg gtg cag agg      48
Met Ala Asp His Leu Met Leu Ala Glu Gly Tyr Arg Leu Val Gln Arg
1               5                   10                  15

ccg ccg tcc gcc gcg gcc gcc cat ggc cct cat gcg ctc cgg act ctg      96
Pro Pro Ser Ala Ala Ala Ala His Gly Pro His Ala Leu Arg Thr Leu
            20                  25                  30

ccg ccg tac gcg ggc ccg ggc ctg gac agt ggg ctg agg ccg cgg ggg      144
Pro Pro Tyr Ala Gly Pro Gly Leu Asp Ser Gly Leu Arg Pro Arg Gly
        35                  40                  45

gct ccg ctg ggg ccg ccg ccg ccc cgc caa ccc ggg gcc ctg gcg tac      192
Ala Pro Leu Gly Pro Pro Pro Pro Arg Gln Pro Gly Ala Leu Ala Tyr
    50                  55                  60

ggg gcc ttc ggg ccg ccg tcc tcc ttc cag ccc ttt ccg gcc gtg cct      240
Gly Ala Phe Gly Pro Pro Ser Ser Phe Gln Pro Phe Pro Ala Val Pro
65                  70                  75                  80

ccg ccg gcc gcg ggc atc gcg cac ctg cag cct gtg gcg acg ccg tac      288
Pro Pro Ala Ala Gly Ile Ala His Leu Gln Pro Val Ala Thr Pro Tyr
                85                  90                  95

ccc ggc cgc gcg gcc gcg ccc ccc aac gct ccg gga ggc ccc ccg ggc      336
Pro Gly Arg Ala Ala Ala Pro Pro Asn Ala Pro Gly Gly Pro Pro Gly
            100                 105                 110

ccg cag ccg gcg cca agc gcc gca gcc ccg ccg ccg ccc gcg cac gcc      384
Pro Gln Pro Ala Pro Ser Ala Ala Ala Pro Pro Pro Pro Ala His Ala
        115                 120                 125

ctg ggc ggc atg gac gcc gaa ctc atc gac gag gag gcg ctg acg tcg      432
Leu Gly Gly Met Asp Ala Glu Leu Ile Asp Glu Glu Ala Leu Thr Ser
    130                 135                 140

ctg gag ctg gag ctc ggg ctg cac cgc gtg cgc gag ctg ccc gag ctc      480
Leu Glu Leu Glu Leu Gly Leu His Arg Val Arg Glu Leu Pro Glu Leu
145                 150                 155                 160

ttc ctg ggc cag agc gag ttc gac tgc ttc tcg gac ttg ggg tcc gcg      528

Phe Leu Gly Gln Ser Glu Phe Asp Cys Phe Ser Asp Leu Gly Ser Ala
                165                 170                 175

ccg ccc gcc ggc tcc gtg agc tgc                                   552
Pro Pro Ala Gly Ser Val Ser Cys
            180

<210>  2
<211>  184
<212>  PRT
<213>  Homo sapiens

<220>
<221>  misc_feature
<223>  Description of sequence: amino acid sequence of CITED4

<400>  2

Met Ala Asp His Leu Met Leu Ala Glu Gly Tyr Arg Leu Val Gln Arg
1               5                   10                  15

Pro Pro Ser Ala Ala Ala Ala His Gly Pro His Ala Leu Arg Thr Leu
            20                  25                  30

Pro Pro Tyr Ala Gly Pro Gly Leu Asp Ser Gly Leu Arg Pro Arg Gly
        35                  40                  45

Ala Pro Leu Gly Pro Pro Pro Pro Arg Gln Pro Gly Ala Leu Ala Tyr
    50                  55                  60

Gly Ala Phe Gly Pro Pro Ser Ser Phe Gln Pro Phe Pro Ala Val Pro
65                  70                  75                  80

Pro Pro Ala Ala Gly Ile Ala His Leu Gln Pro Val Ala Thr Pro Tyr
                85                  90                  95

Pro Gly Arg Ala Ala Ala Pro Pro Asn Ala Pro Gly Gly Pro Pro Gly
            100                 105                 110

Pro Gln Pro Ala Pro Ser Ala Ala Ala Pro Pro Pro Pro Ala His Ala
        115                 120                 125

Leu Gly Gly Met Asp Ala Glu Leu Ile Asp Glu Glu Ala Leu Thr Ser
    130                 135                 140

Leu Glu Leu Glu Leu Gly Leu His Arg Val Arg Glu Leu Pro Glu Leu
145                 150                 155                 160

Phe Leu Gly Gln Ser Glu Phe Asp Cys Phe Ser Asp Leu Gly Ser Ala
                165                 170                 175

Pro Pro Ala Gly Ser Val Ser Cys
            180

<210>  3
<211>  20
<212>  DNA
<213>  Artificial

<220>
<223>  Description of sequence: Primer CITED4-P1-for

<400>  3
gggccaagac ctagatgcag                                                    20


<210>  4
<211>  19
<212>  DNA
<213>  Artificial

<220>
<223>  Description of sequence:  Primer CITED4-P1-rev

<400>  4
aaaccaaacc cgactggtg                                                     19


<210>  5
<211>  18
<212>  DNA
<213>  Artificial

<220>
<223>  Description of sequence: Primer CITED4-P2-for

<400>  5
cgcaaggtgc gcagtagt                                                      18


<210>  6
<211>  18
<212>  DNA
<213>  Artificial

<220>
<223>  Description of sequence: Primer CITED4-P2-rev

<400>  6
ctctgcacca ggcggtag                                                      18


<210>  7
<211>  18
<212>  DNA
<213>  Artificial

<220>
<223>  Description of sequence: Primer CITED4-P3-for

<400>  7
catggccgac cacctgat                                                      18


<210>  8
<211>  20
<212>  DNA
<213>  Artificial

<220>
<223>  Description of sequence: Primer CITED4-P3-rev

<400>  8
gaaagggctg gaaggaggac                                                    20


<210>  9
<211>  18
<212>  DNA
<213>  Artificial

<220>
<223>  Description of sequence: Primer CITED4-P4-for

<400>  9
ctaccgcctg gtgcagag                                                      18


<210>  10
<211>  20
<212>  DNA
<213>  Artificial

<220>
<223>  Description of sequence: Primer CITED4-P4-rev

<400>  10
agtccgagaa gcagtcgaac                                                    20


<210>  11
<211>  19
<212>  DNA
<213>  Artificial

<220>
<223>  Description of sequence: Primer CITED4-P5-for

<400>  11
catggacgcc gaactcatc                                                     19


<210>  12
<211>  20
<212>  DNA
<213>  Artificial

<220>
<223>  Description of sequence: Primer CITED4-P5-rev

<400>  12
agtcgggccc tttctcctct                                                    20


<210>  13
<211>  20
<212>  DNA
<213>  Artificial

<220>
<223>  Description of sequence: Primer CITED4-P6-for

<400>  13
gggaggacag tttggcttca                                                    20


<210>  14
<211>  20
<212>  DNA
<213>  Artificial

<220>
<223>  Description of sequence: Primer CITED4-P6-rev

<400>  14
gggagaggac acgatccaag                                                    20


<210>  15
<211>  27
<212>  DNA
<213>  Artificial

<220>
<223>  Description of sequence: Primer CITED4-P7-for

<400>  15
tttggttagt ttaagtttta ttttagt                                            27


<210>  16
<211>  25
<212>  DNA
<213>  Artificial

<220>
<223>  Description of sequence: Primer CITED4-P7-rev

<400>  16
aatcacaaaa acccgcaacc tataa                                              25


<210>  17
<211>  25
<212>  DNA
<213>  Artificial

<220>
<223>  Description of sequence: Primer CITED4-P8-for

<400>  17
ttataggttg cgggtttttg tgatt                                              25


<210>  18
<211>  22
<212>  DNA
<213>  Artificial

<220>
<223>  Description of sequence: Primer CITED4-P8-rev

<400>  18
acctctacac caaacgataa cc                                                 22


<210>  19
<211>  35
<212>  DNA
<213>  Artificial

<220>
<223>  Description of sequence:  Primer CITED4-Met-F1

<400>  19
gcggccgctt ttttgggata gaatttaaat agttt                                   35

<210> 20
<211> 24
<212> DNA
<213> Artificial

<220>
<223> Description of sequence: Primer CITED4-Met-R1

<400> 20
ccaaactaaa ctacaataac gcta 24


<210> 21
<211> 35
<212> DNA
<213> Artificial

<220>
<223> Description of sequence: CITED4-Met-F2

<400> 21
gcggccgctt tggttagttt aagttttatt ttagt 35


<210> 22
<211> 25
<212> DNA
<213> Artificial

<220>
<223> Description of sequence: Primer CITED4-Met-R2

<400> 22
aatcacaaaa acccgcaacc tataa 25


<210> 23
<211> 33
<212> DNA
<213> Artificial

<220>
<223> Description of sequence: CITED4-Met-F3

<400> 23
gcggccgctt ataggttgcg ggtttttgtg att 33


<210> 24
<211> 22
<212> DNA
<213> Artificial

<220>
<223> Description of sequence: Primer CITED4-Met-R3

<400> 24
acctctacac caaacgataa cc 22


<210> 25
<211> 23
<212> DNA
<213> Artificial

<220>
<223> Description of sequence: Primer CITED4MSP-metF

<400>  25
cgcggttcgt ttagaagcgt ttc                                                23

<210>  26
<211>  21
<212>  DNA
<213>  Artificial

<220>
<223>  Description of sequence: Primer CITED4MSP-metR1

<400>  26
cgcgtcgcgt aactaaactc g                                                  21

<210>  27
<211>  18
<212>  DNA
<213>  Artificial

<220>
<223>  Description of sequence: Primer CITED4MSP-metR2

<400>  27
cgaaacctcc gcgtcgcg                                                      18

<210>  28
<211>  35
<212>  DNA
<213>  Artificial

<220>
<223>  Description of sequence: Primer CITED4MSP-F

<400>  28
tgtggtttgt ttagaagtgt tttgtttagt taatg                                   35

<210>  29
<211>  34
<212>  DNA
<213>  Artificial

<220>
<223>  Description of sequence: Primer CITED4MSP-R1

<400>  29
cacatcacat aactaaactc aacatattct tata                                    34

<210>  30
<211>  30
<212>  DNA
<213>  Artificial

<220>
<223>  Description of sequence:  Primer CITED4MSP-R2

<400>  30
caaaacctcc acatcacata actaaactca                                         30

<210>  31
<211>  33
<212>  DNA
<213>  Artificial

<220>
<223>  Description of sequence: Primer CITED4BisF2gegen

<400>  31
gcggccgcgg ttataaaggt tcgtaatttg tag                                    33


<210>  32
<211>  27
<212>  DNA
<213>  Artificial

<220>
<223>  Description of sequence: Primer CITED4BisR2gegen

<400>  32
tctaatcaat tcaaactcta ccccaac                                           27


<210>  33
<211>  29
<212>  DNA
<213>  Artificial

<220>
<223>  Description of sequence: Primer CITED4-Met-F4

<400>  33
gcggccgcgg gttaagattt agatgtagg                                         29


<210>  34
<211>  27
<212>  DNA
<213>  Artificial

<220>
<223>  Description of sequence: CITED4BisR4gegen

<400>  34
aaaacaaaac caaaacctaa atacaaa                                           27


<210>  35
<211>  20
<212>  DNA
<213>  Artificial

<220>
<223>  Description of sequence: Primer ARF1-P1-for

<400>  35
gaccacgatc ctctacaagc                                                   20


<210>  36
<211>  21
<212>  DNA
<213>  Artificial

<220>
<223>  Description of sequence: Primer ARF1-P1-rev

<400>  36
tcccacacag tgaagctgat g                                                 21

<210> 37
<211> 21
<212> DNA
<213> Artificial

<220>
<223> Description of sequence: Primer ACTG1-P1-for

<400> 37
cagctctcgc actctgttct t 21

<210> 38
<211> 19
<212> DNA
<213> Artificial

<220>
<223> Description of sequence. Primer ACTG1-P1-rev

<400> 38
cgacgatgga aggaaacac 19

<210> 39
<211> 2536
<212> DNA
<213> Homo sapiens

<220>
<221> misc_feature
<223> Description of sequence: non-methylated CITED 4 gene including 5'
and 3' regulatory sequences (chr1: 40995843 - 40997578 (opposite
strand)) (non-methylated)

<220>
<221> misc_feature
<222> (1)..(1000)
<223> 5'-upstream sequence of CITED4

<220>
<221> misc_feature
<222> (1268)..(2536)
<223> cDNA-Sequenz CITED4

<220>
<221> CDS
<222> (1487)..(2041)

<220>
<221> misc_feature
<222> (2336)..(2536)
<223> 3'-downstream sequence of CITED4

<400> 39
gcactttaaa agtccaagtt attattgctg ttatcaagag aaggcccttt gggacagaac 60

ttaaatagcc ttggccgggc gcggtggctc acgcctgtaa ttccagcact ttgggaggcc 120

gaggcgggcg gatcacgagg tcagcagatc gagaccatcc tggctaacac ggtgaaaccc 180

cgtctctact aaaaatacaa aaaaaattag ccgggcgtgg tagcgggcgc ctgtagtccc 240

agctactcgg gagactgagg caggagaatg gcgtgaaccc gggaggcgga gcttgcagtg 300

agccaagaca gcgccactgc agtccagcct gggcgaaaga gcgagactcc gtctcaaaaa 360

aaaaaaaaaa aagaacttaa atagcctttc tttcatctaa gggacacttt tgagcatcta 420

ccacatacca ggcactgtgc taggcactgt gcacacatgc cctcacttac ccctcacaaa 480

gggtcagtgc tgctgttctt taccttttac agatgaagaa actgaggctc agctgagagt 540

tgcaattcat tccatcatta gttcattcac tcagctttta ctaaaagcct gctctgggcc 600

aggctctggg ccaagtgctg agaagaagaa gatgaacaag tccttcagga gacgtgccca 660

taaattaata acaactaagt acatgcttca acagacaagg tgcagtggga actagagggg 720

gagcaacagg ctctcctggg ggttatggga atggacagct gacctcctgc ggggaccctg 780

caccgctaag gtgcatgcgc cctgtgtagc tgtgcagtct cactttgatc aaggctttgc 840

tgtgtatttc caggaagcct cttccgccac tgagcctcgg tctctacctg gaaagttaaa 900

aggttgcgta ggcctgacat cctgcagctc ttggggggcc taaaactctg gccctcccac 960

cccaccttct ggtcagttca agctctaccc cagccaagtc cgattccgaa gccctgaggg 1020

ccagaccgaa tccctccgaa agtgccaaat accccgcccc agggcggtgc tcagagcctg 1080

gggcgtggcc ggagtcccaa ggggcggggt tccagtggga ggggcggggc caagacctag 1140

atgcaggcgt gcgcggcccg cccagaagcg tctcgcccag ccaatgagcg tccgagggcg 1200

gggaagcccc gcctctgggt ataagaatac gccgagccca gctacgcgac gcggaggttt 1260

cggagcacta caggttgcgg gcctttgtga ccccaggctg cggccgcaag gtgcgcagta 1320

gtgggctggg ccggggcacc agtcgggttt ggttttgctc cgcccggccg agctgcaggg 1380

accgcacggt gcccgggtct cccgccgcag agcagccgct ggcccgggcc gggggagccg 1440

gcgctgacca ccgcgcgctg tccccgcagg cagccggcct gccgcc atg gcc gac 1495
Met Ala Asp
1

cac ctg atg ctc gcc gag ggc tac cgc ctg gtg cag agg ccg ccg tcc 1543
His Leu Met Leu Ala Glu Gly Tyr Arg Leu Val Gln Arg Pro Pro Ser
    5                   10                  15

gcc gcg gcc gcc cat ggc cct cat gcg ctc cgg act ctg ccg ccg tac 1591
Ala Ala Ala Ala His Gly Pro His Ala Leu Arg Thr Leu Pro Pro Tyr
20                  25                  30                  35

gcg ggc ccg ggc ctg gac agt ggg ctg agg ccg cgg ggg gct ccg ctg 1639
Ala Gly Pro Gly Leu Asp Ser Gly Leu Arg Pro Arg Gly Ala Pro Leu
                40                  45                  50

ggg ccg ccg ccg ccc cgc caa ccc ggg gcc ctg gcg tac ggg gcc ttc 1687
Gly Pro Pro Pro Pro Arg Gln Pro Gly Ala Leu Ala Tyr Gly Ala Phe
            55                  60                  65

ggg ccg ccg tcc tcc ttc cag ccc ttt ccg gcc gtg cct ccg ccg gcc 1735
Gly Pro Pro Ser Ser Phe Gln Pro Phe Pro Ala Val Pro Pro Pro Ala
        70                  75                  80

gcg ggc atc gcg cac ctg cag cct gtg gcg acg ccg tac ccc ggc cgc 1783
Ala Gly Ile Ala His Leu Gln Pro Val Ala Thr Pro Tyr Pro Gly Arg
    85                  90                  95

gcg gcc gcg ccc ccc aac gct ccg gga ggc ccc ccg ggc ccg cag ccg 1831
Ala Ala Ala Pro Pro Asn Ala Pro Gly Gly Pro Pro Gly Pro Gln Pro
100                 105                 110                 115

gcg cca agc gcc gca gcc ccg ccg ccg ccc gcg cac gcc ctg ggc ggc     1879
Ala Pro Ser Ala Ala Ala Pro Pro Pro Pro Ala His Ala Leu Gly Gly
            120                 125                 130

atg gac gcc gaa ctc atc gac gag gag gcg ctg acg tcg ctg gag ctg     1927
Met Asp Ala Glu Leu Ile Asp Glu Glu Ala Leu Thr Ser Leu Glu Leu
            135                 140                 145

gag ctc ggg ctg cac cgc gtg cgc gag ctg ccc gag ctc ttc ctg ggc     1975
Glu Leu Gly Leu His Arg Val Arg Glu Leu Pro Glu Leu Phe Leu Gly
        150                 155                 160

cag agc gag ttc gac tgc ttc tcg gac ttg ggg tcc gcg ccg ccc gcc     2023
Gln Ser Glu Phe Asp Cys Phe Ser Asp Leu Gly Ser Ala Pro Pro Ala
    165                 170                 175

ggc tcc gtg agc tgc tga gggcggccgg cgcccgcccg gcgtgccgga            2071
Gly Ser Val Ser Cys
180

gaggagaaag ggcccgactg cccgccggac cctgcaccca gcgactgggc cccgcgcgcg   2131

ccctccgcga gggtggaggc ggcggctgtg tgcgcagggc ccggcaccgg actgggaccc   2191

tggcgtccct ccaggccttg cctcctgcgg gaggacagtt tggcttcact tctctgaccc   2251

cagcctcggc cgtaaagtga aagagaccgg accagcttca gctttcggac tctggttctt   2311

ggatcgtgtc ctctccccct cgccgccctc ttcccccaat ctgagccatt gcaggcctct   2371

gcctgctgcc ccctctctcc tcgggatcgg gtccccagag ccaccatctc ctgagcctcc   2431

caccccgctg cctgggccct gtggttgctg ggcctcccac ctcaaggagg ggaaggttgt   2491

acagcccgaa cccgtggagc aatgccctgt ctggcctcca aaacc                   2536

<210>  40
<211>  2536
<212>  DNA
<213>  Homo sapiens

<220>
<221>  misc_feature
<223>  Description of sequence:  methylated CITED 4 gene including 5'
       and 3' regulatory sequences (chr1: 40995843 - 40997578 (opposite
       strand)) after bisulfite-treatment

<400>  40
gtattttaaa agtttaagtt attattgttg ttattaagag aaggtttttt gggatagaat     60

ttaaatagtt ttggtcgggc gcggtggttt acgtttgtaa ttttagtatt ttgggaggtc    120

gaggcgggcg gattacgagg ttagtagatc gagattattt tggttaatac ggtgaaattt    180

cgtttttatt aaaaatataa aaaaaattag tcgggcgtgg tagcgggcgt ttgtagtttt    240

agttattcgg gagattgagg taggagaatg gcgtgaattc gggaggcgga gtttgtagtg    300

agttaagata gcgttattgt agtttagttt gggcgaaaga gcgagatttc gttttaaaaa    360

aaaaaaaaaa aagaatttaa atagtttttt ttttatttaa gggatatttt tgagtattta    420

ttatatatta ggtattgtgt taggtattgt gtatatatgt ttttatttat tttttataaa    480

gggttagtgt tgttgttttt tattttttat agatgaagaa attgaggttt agttgagagt    540

tgtaatttat tttattatta gtttatttat ttagttttta ttaaaagttt gttttgggtt 600
aggttttggg ttaagtgttg agaagaagaa gatgaataag ttttttagga gacgtgttta 660
taaattaata ataattaagt atatgtttta atagataagg tgtagtggga attagagggg 720
gagtaatagg tttttttggg ggttatggga atggatagtt gattttttgc ggggattttg 780
tatcgttaag gtgtatgcgt tttgtgtagt tgtgtagttt tattttgatt aaggttttgt 840
tgtgtatttt taggaagttt ttttcgttat tgagtttcgg tttttatttg gaaagttaaa 900
aggttgcgta ggtttgatat tttgtagttt ttggggggtt taaaattttg gtttttttat 960
tttatttttt ggttagttta agttttattt tagttaagtt cgatttcgaa gttttgaggg 1020
ttagatcgaa ttttttcgaa agtgttaaat atttcgtttt agggcggtgt ttagagtttg 1080
gggcgtggtc ggagttttaa ggggcggggt tttagtggga ggggcggggt taagatttag 1140
atgtaggcgt gcgcggttcg tttagaagcg tttcgtttag ttaatgagcg ttcgagggcg 1200
gggaagtttc gtttttgggt ataagaatac gtcgagttta gttacgcgac gcggaggttt 1260
cggagtatta taggttgcgg gtttttgtga ttttaggttg cggtcgtaag gtgcgtagta 1320
gtgggttggg tcggggtatt agtcgggttt ggttttgttt cgttcggtcg agttgtaggg 1380
atcgtacggt gttcgggttt ttcgtcgtag agtagtcgtt ggttcgggtc gggggagtcg 1440
gcgttgatta tcgcgcgttg ttttcgtagg tagtcggttt gtcgttatgg tcgattattt 1500
gatgttcgtc gagggttatc gtttggtgta gaggtcgtcg ttcgtcgcgg tcgtttatgg 1560
tttttatgcg tttcggattt tgtcgtcgta cgcgggttcg ggtttggata gtgggttgag 1620
gtcgcggggg gtttcgttgg ggtcgtcgtc gtttcgttaa ttcggggttt tggcgtacgg 1680
ggttttcggg tcgtcgtttt ttttttagtt tttttcggtc gtgttttcgt cggtcgcggg 1740
tatcgcgtat ttgtagtttg tggcgacgtc gtatttcggt cgcgcggtcg cgttttttaa 1800
cgtttcggga ggtttttcgg gttcgtagtc ggcgttaagc gtcgtagttt cgtcgtcgtt 1860
cgcgtacgtt ttgggcggta tggacgtcga atttatcgac gaggaggcgt tgacgtcgtt 1920
ggagttggag ttcgggttgt atcgcgtgcg cgagttgttc gagttttttt tgggttagag 1980
cgagttcgat tgtttttcgg atttggggtt cgcgtcgttc gtcggtttcg tgagttgttg 2040
agggcggtcg gcgttcgttc ggcgtgtcgg agaggagaaa gggttcgatt gttcgtcgga 2100
ttttgtattt agcgattggg tttcgcgcgc gtttttcgcg agggtggagg cggcggttgt 2160
gtgcgtaggg ttcggtatcg gattgggatt ttggcgtttt tttaggtttt gttttttgcg 2220
ggaggatagt ttggttttat ttttttgatt ttagtttcgg tcgtaaagtg aaagagatcg 2280
gattagtttt agttttcgga ttttggtttt tggatcgtgt tttttttttt tcgtcgtttt 2340
ttttttttaa tttgagttat tgtaggtttt tgtttgttgt tttttttttt ttcgggatcg 2400
ggtttttaga gttattattt tttgagtttt ttatttcgtt gtttgggttt tgtggttgtt 2460
gggtttttta ttttaaggag gggaaggttg tatagttcga attcgtggag taatgttttg 2520
tttggttttt aaaatt 2536

<210>  41
<211>  2536
<212>  DNA
<213>  Homo sapiens


<220>
<221>  misc_feature
<223>  Description of sequence:  reverse non-methylated  CITED 4 gene
       including 5' and 3' regulatory sequences (chr1: 40995843 -
       40997578 (opposite strand))

<400>  41
ggttttggag gccagacagg gcattgctcc acgggttcgg gctgtacaac cttcccctcc      60

ttgaggtggg aggcccagca accacagggc ccaggcagcg gggtgggagg ctcaggagat     120

ggtggctctg gggacccgat cccgaggaga gagggggcag caggcagagg cctgcaatgg     180

ctcagattgg gggaagaggg cggcgagggg gagaggacac gatccaagaa ccagagtccg     240

aaagctgaag ctggtccggt ctctttcact ttacggccga ggctggggtc agagaagtga     300

agccaaactg tcctcccgca ggaggcaagg cctggaggga cgccagggtc ccagtccggt     360

gccgggccct gcgcacacag ccgccgcctc caccctcgcg gagggcgcgc gcggggccca     420

gtcgctgggt gcagggtccg gcgggcagtc gggccctttc tcctctccgg cacgccgggc     480

gggcgccggc cgccctcagc agctcacgga gccggcgggc ggcgcggacc ccaagtccga     540

gaagcagtcg aactcgctct ggcccaggaa gagctcgggc agctcgcgca cgcggtgcag     600

cccgagctcc agctccagcg acgtcagcgc ctcctcgtcg atgagttcgg cgtccatgcc     660

gcccagggcg tgcgcgggcg gcggcggggc tgcggcgctt ggcgccggct gcgggcccgg     720

ggggcctccc ggagcgttgg ggggcgcggc cgcgcggccg gggtacggcg tcgccacagg     780

ctgcaggtgc gcgatgcccg cggccggcgg aggcacggcc ggaaagggct ggaaggagga     840

cggcggcccg aaggccccgt acgccagggc cccgggttgg cggggcggcg gcggccccag     900

cggagccccc cgcggcctca gcccactgtc caggcccggg cccgcgtacg gcggcagagt     960

ccggagcgca tgagggccat gggcggccgc ggcggacggc ggcctctgca ccaggcggta    1020

gccctcggcg agcatcaggt ggtcggccat ggcggcaggc cggctgcctg cggggacagc    1080

gcgcggtggt cagcgccggc tcccccggcc cgggccagcg gctgctctgc ggcgggagac    1140

ccgggcaccg tgcggtccct gcagctcggc cgggcggagc aaaaccaaac ccgactggtg    1200

ccccggccca gcccactact gcgcaccttg cggccgcagc ctggggtcac aaaggcccgc    1260

aacctgtagt gctccgaaac ctccgcgtcg cgtagctggg ctcggcgtat tcttataccc    1320

agaggcgggg cttccccgcc ctcggacgct cattggctgg gcgagacgct tctgggcggg    1380

ccgcgcacgc ctgcatctag gtcttggccc cgcccctccc actggaaccc cgccccttgg    1440

gactccggcc acgccccagg ctctgagcac cgccctgggg cggggtattt ggcactttcg    1500

gagggattcg gtctggccct cagggcttcg gaatcggact tggctggggt agagcttgaa    1560

ctgaccagaa ggtggggtgg gagggccaga gttttaggcc ccccaagagc tgcaggatgt    1620

caggcctacg caacctttta actttccagg tagagaccga ggctcagtgg cggaagaggc 1680

ttcctggaaa tacacagcaa agccttgatc aaagtgagac tgcacagcta cacagggcgc 1740

atgcacctta gcggtgcagg gtccccgcag gaggtcagct gtccattccc ataaccccca 1800

ggagagcctg ttgctccccc tctagttccc actgcacctt gtctgttgaa gcatgtactt 1860

agttgttatt aatttatggg cacgtctcct gaaggacttg ttcatcttct tcttctcagc 1920

acttggccca gagcctggcc cagagcaggc ttttagtaaa agctgagtga atgaactaat 1980

gatggaatga attgcaactc tcagctgagc ctcagtttct tcatctgtaa aaggtaaaga 2040

acagcagcac tgaccctttg tgaggggtaa gtgagggcat gtgtgcacag tgcctagcac 2100

agtgcctggt atgtggtaga tgctcaaaag tgtcccttag atgaaagaaa ggctatttaa 2160

gttctttttt tttttttttt tgagacggag tctcgctctt tcgcccaggc tggactgcag 2220

tggcgctgtc ttggctcact gcaagctccg cctcccgggt tcacgccatt ctcctgcctc 2280

agtctcccga gtagctggga ctacaggcgc ccgctaccac gcccggctaa tttttttgt 2340

atttttagta gagacggggt ttcaccgtgt tagccaggat ggtctcgatc tgctgacctc 2400

gtgatccgcc cgcctcggcc tcccaaagtg ctggaattac aggcgtgagc caccgcgccc 2460

ggccaaggct atttaagttc tgtcccaaag ggccttctct tgataacagc aataataact 2520

tggactttta aagtgc 2536

<210> 42
<211> 2536
<212> DNA
<213> Homo sapiens

<220>
<221> misc_feature
<223> Description of sequence: reverse methylated CITED 4 gene
including 5' and 3' regulatory sequences (chr1: 40995843 -
40997578 (opposite strand)) after bisulfite-treatment

<400> 42
   ggttttggag gttagatagg gtattgtttt acgggttcgg gttgtataat tttttttttt 60
   ttgaggtggg aggtttagta attatagggt ttaggtagcg gggtgggagg tttaggagat 120
   ggtggttttg gggattcgat ttcgaggaga gagggggtag taggtagagg tttgtaatgg 180
   tttagattgg gggaagaggg cggcgagggg gagaggatac gatttaagaa ttagagttcg 240
   aaagttgaag ttggttcggt tttttttatt ttacggtcga ggttggggtt agagaagtga 300
   agttaaattg ttttttcgta ggaggtaagg tttggaggga cgttagggtt ttagttcggt 360
   gtcgggtttt gcgtatatag tcgtcgtttt tattttcgcg gagggcgcgc gcggggttta 420
   gtcgttgggt gtagggttcg gcgggtagtc gggttttttt tttttttcgg tacgtcgggc 480
   gggcgtcggt cgtttttagt agtttacgga gtcggcgggc ggcgcggatt ttaagttcga 540
   gaagtagtcg aattcgtttt ggtttaggaa gagttcgggt agttcgcgta cgcggtgtag 600
   ttcgagtttt agttttagcg acgttagcgt tttttcgtcg atgagttcgg cgtttatgtc 660
   gtttagggcg tgcgcgggcg gcggcggggt tgcggcgttt ggcgtcggtt gcgggttcgg 720
   ggggtttttc ggagcgttgg ggggcgcggt cgcgcggtcg gggtacggcg tcgttatagg 780
   ttgtaggtgc gcgatgttcg cggtcggcgg aggtacggtc ggaaagggtt ggaaggagga 840
   cggcggttcg aaggtttcgt acgttagggt ttcgggttgg cggggcggcg gcggttttag 900
   cggagttttt cgcggtttta gtttattgtt taggttcggg ttcgcgtacg gcggtagagt 960
   tcggagcgta tgagggttat gggcggtcgc ggcggacggc ggtttttgta ttaggcggta 1020
   gttttcggcg agtattaggt ggtcggttat ggcggtaggt cggttgtttg cggggatagc 1080
   gcgcggtggt tagcgtcggt tttttcggtt cgggttagcg gttgttttgc ggcgggagat 1140
   tcgggtatcg tgcggttttt gtagttcggt cgggcggagt aaaattaaat tcgattggtg 1200
   tttcggttta gtttattatt gcgtattttg cggtcgtagt ttggggttat aaaggttcgt 1260
   aatttgtagt gtttcgaaat tttcgcgtcg cgtagttggg ttcggcgtat ttttatattt 1320
   agaggcgggg ttttttcgtt ttcggacgtt tattggttgg gcgagacgtt tttgggcggg 1380
   tcgcgtacgt ttgtatttag gttttggttt cgtttttttt attggaattt cgttttttgg 1440
   gatttcggtt acgttttagg ttttgagtat cgttttgggg cggggtattt ggtattttcg 1500
   gagggattcg gtttggtttt tagggtttcg gaatcggatt tggttggggt agagtttgaa 1560
   ttgattagaa ggtggggtgg gagggttaga gttttaggtt ttttaagagt tgtaggatgt 1620
   taggtttacg taatttttta attttttagg tagagatcga ggtttagtgg cggaagaggt 1680
   tttttggaaa tatatagtaa agttttgatt aaagtgagat tgtatagtta tatagggcgt 1740
   atgtatttta gcggtgtagg gttttcgtag gaggttagtt gtttattttt ataattttta 1800
   ggagagtttg ttgttttttt tttagttttt attgtatttt gtttgttgaa gtatgtattt 1860
   agttgttatt aatttatggg tacgtttttt gaaggatttg tttatttttt tttttttagt 1920
   atttggttta gagtttggtt tagagtaggt ttttagtaaa agttgagtga atgaattaat 1980
   gatggaatga attgtaattt ttagttgagt tttagttttt ttatttgtaa aaggtaaaga 2040
   atagtagtat tgattttttg tgaggggtaa gtgagggtat gtgtgtatag tgtttagtat 2100
   agtgtttggt atgtggtaga tgtttaaaag tgttttttag atgaaagaaa ggttatttaa 2160
   gttttttttt tttttttttt tgagacggag tttcgttttt tcgtttaggt tggattgtag 2220
   tggcgttgtt ttggtttatt gtaagtttcg tttttcgggt ttacgttatt tttttgtttt 2280
   agtttttcga gtagttggga ttataggcgt tcgttattac gttcggttaa ttttttttgt 2340
   atttttagta gagacggggt tttatcgtgt tagttaggat ggtttcgatt tgttgatttc 2400
   gtgattcgtt cgtttcggtt ttttaaagtg ttggaattat aggcgtgagt tatcgcgttc 2460
   ggttaaggtt atttaagttt tgttttaaag ggtttttttt tgataatagt aataataatt 2520
   tggattttta aagtgt 2536

## Description

The present invention provides novel (diagnostic) methods for detecting cancers of the central nervous system, preferably oligodendrogliomas, by detecting the presence of CITED4-nucleic acid sequences in a biological sample. The present invention furthermore provides a (diagnostic) method for determining the degree of methylation of the promoter region of the CITED4 gene in a biological sample, preferably derived from cancers of the central nervous system including oligodendrogliomas. As a further embodiment, a method for gene-therapy suitable for treating or preventing cancers of the central nervous system, preferably oligodendrogliomas, is provided. Finally, nucleic acid sequences, amino acid sequences, vectors, antibodies, pharmaceutical compositions and kits, suitable for these inventive methods are disclosed herein.

Cancer, in particular cancer of the central nervous system (CNS), is a complex disease and, despite of improved treatments, it still represents a leading cause of death in the world. Furthermore, the prognosis for most patients with cancer of the central nervous system, particular with brain tumors, remains poor. Therefore, many attempts have been made to provide efficient methods of therapy and diagnosis for such diseases. Since the prospect of complete remission of cancer is, in most cases, greatly enhanced by diagnosis prior to or concomitant to therapy, it is highly desirable to detect cancer before a substantial tumor develops. Also, in cases where the primary tumor has been substantially removed by surgery or destroyed by other means, it is important that any remainder of the cancer in the patient, either in the form of residues of the primary tumor or of secondary tumors caused by metastasis may be detected, in order to prescribe an appropriate subsequent treatment, such as e.g. a post-operative chemotherapy. Thus, the establishment of tumor grade is a key determinant in the choice of a therapeutic approach and in prognosis.

Among various types of cancer, particularly among cancers of the central nervous system, glioma represent the most common primary (brain) tumors, arising from glial cells like astrocytic and oligodendroglial cells. They show wide diversity with respect to location, morphology, genetic status and response to therapy. Oligodendroglioma are classified as WHO grade II glioma (Kleihues, P. et al., J Neuropathol Exp Neurol 2002;61:215-25) and the more malignant anaplastic oligodendroglioma is classified as WHO grade III glioma. Nuclear atypia and occasional mitosis can be seen in a WHO grade II oligodendroglioma, marked mitotic activity, noticeable microvascular proliferation and additional or exclusive necrosis are hallmarks of anaplastic oligodendroglioma (Engelhard, H.H. et al., (2002) Surg Neurol, 58, 111-7; discussion 117; Radner, H. et al, (2002), Pathologe, 23, 260-83). Mixed glioma (oligoastrocytoma) contain both, neoplastic astrocytes as well as neoplastic oligodendroglial cells. Oligodendroglial tumors, including oligodendrogliomas and mixed oligoastrocytomas, are primary tumors of the central nervous system that account for 5-18% of all gliomas.

Tumors of the central nervous system may be detected by using various biochemical and immunological entities as markers to indicate the presence, nature or extent of these cancers in mammals. Typical marker molecules produced by cancers include e.g. monoclonal immunoglobulins, hormones, secreted serum proteins, antigens, enzymes and isoenzymes, cell surface markers, glycoproteins and carbohydrates, extracellular matrix proteins and mucins. These cancer markers may be categorized as soluble markers, which appear in body fluids such as blood, plasma, serum, effusions, and urine. Other cancer markers are either cell-associated proteins or nucleic acids, wherein both of them are characteristically not released into serum or other body fluids in significant amounts. Cell- or tissue-associated tumor markers are detectable in tissue samples containing cancerous cells, or in biological samples that contain such cells (e.g. biological samples containing brain cells). Unfortunately, many of the presently known cancer markers are only applicable to a narrow range of cancer types but not for tumors of the central nervous system, e.g. brain tumors, including gliomas such as oligodendroglial tumors.

Apart from those cancer markers discussed above, cancer markers may be "oncodevelopmental" markers which reflect the (expression) state of cancer cells (compared to "non-cancerous" cells), or which reflect molecular changes in cancerogenic cells due to activation of cell division pathways, inhibition of cell death pathways or deletion or inactivation of genes, e.g. by "loss of heterozygosity" (LOH). Loss of heterozygosity in cancer cells usually occurs due to loss of one allele in a locus which is heterozygous in the individual's normal cells. This phenomenon is particularly relevant for involvement of dysfunctional tumor suppressor genes in tumor development. Because tumor suppressor genes are recessive, cells containing a normal and a mutated gene - i.e. are heterozygous - still behave normally. However, there are several mechanisms which may cause a cell to lose its functional allele. Thus, these cells are predisposed to develop into a tumor. By way of example, mechanisms of LOH are (i) deletion of the normal allele; (ii) deletion of the chromosome arm containing the normal allele; (iii) deletion of the entire chromosome containing the normal allele (resulting in aneuploidy), (iv) loss of the chromosome containing the normal allele followed by duplication of the chromosome containing the mutated allele or, finally, (v) mitotic recombination, since the study of tumor suppressor genes revealed (for the first time) that crossing over - with genetic recombination - occasionally occurs also in mitosis.

Apart from involvement of dysfunctional tumor suppressor genes in tumor development, LOH, as mentioned above, is detected in most oligodendrogliomas, i.e. by (a) determining the presence of heterozygosity, e.g. the occurrence of different alleles, at a genetic locus in an organism's germline DNA derived from tumor free tissue, and (b) determining the absence of heterozygosity at that particular locus in the individual's cancer cells. Determination of LOH is usually carried out by using polymorphic markers, such as microsatellites or single nucleotide polymorphisms, for which the two parents contributed different alleles. The majority of oligodendrogliomas and approximately one half of oligoastrocytomas demonstrate such a LOH at polymorphic markers on chromosome arms 1p and 19q (Reifenberger and Louis, J Neuropathol Exp Neurol 2003; 62:111-26.). This aberration has been associated with a favorable response to radio- and chemotherapy as well as prolonged survival (Cairncross, J.G. et al., J Natl Cancer Inst 1998;90:1473-9; Felsberg J. et al., Brain Pathol 2004;14:121-30.). Thus, diagnostic testing for LOH at chromosomal sites 1p/19q may be used to expand conventional histologic tumor classification in the diagnostic and prognostic assessment of oligodendroglial neoplasms (Reifenberger and Louis, J Neuropathol Exp Neurol 2003;62:111-26.). According to former studies, incidence of LOH on 19q varies from 50% to more than 80% in patients suffering from oligodendroglial tumors (Jeuken, J.W. et al., (1999) J Neuropathol Exp Neurol, 58, 606-12; Smith, J.S. et al., (2000) J Clin Oncol, 18, 636-45), while incidence of LOH on 1 p has been reported to occur in 40 to 92% of the patients (Reifenberger, J. et al., (1994) Am J Pathol, 145, 1175-90. ; Smith *et al*., 2000, supra).

Although several genes on 1p and 19q region have been suggested as glioma-associated genes, there is no evidence for their etiological role in oligodendroglial tumors. For example, the p190RhoGAP gene at 19q13.3, which encodes a regulator of Rho kinase, was reported to inhibit PDGF-induced murine oligodendrogliomas (Wolf, R.M. et al., Genes Dev, 2003;17:476-87.). However, aberrations of p190RhoGAP in human oligodendrogliomas have not been reported so far. More recently, the myelin-related epithelial membrane protein gene 3 (EMP3, 19q13.3) was found to be epigenetically silenced in gliomas, including both oligodendroglial and astrocytic tumors (Alaminos M. et a/., Cancer Res 2005;65:2565-71). Candidate glioma relevant genes on 1p include the cyclin-dependent kinase inhibitor 2C (CDKN2C) gene at 1 p32, which is altered in a minor subset of anaplastic oligodendrogliomas (Husemann, K. et al., J Neuropathol Exp Neurol 1999;58:1041-50 ; Pohl, U. et al., Brain Pathol 1999;9:639-43), and the calmodulin-binding transcription activator 1 gene (CAMTA1) at 1p36, which shows reduced expression (but no mutations) in oligodendrogliomas with 1 p deletion (Barbashina, V. et al., Clin Cancer Res 2005;11:1119-28). However, none of these genes proved to be a useful marker suitable in tumors of the central nervous system including oligodendrogliomas.

Summarizing the above, there are presently no cancer markers available in the art, which allow to unequivocally diagnose qualitatively and/or quantitatively the occurrence (as well as the amount) of tumors of the central nervous system including oligodendroglial tumors by molecular biological and/or biophysical methods. Additionally, no efficient methods are yet known, which allow to treat such tumors without applying invasive surgery or chemotherapy.

Thus, it is a first object of the present invention to provide a marker system, which allows to efficiently distinguish between patients with good versus bad prognosis concerning treatment of tumors of the central nervous system. Furthermore, it is a second object to establish a marker system, which efficiently allows to distinguish cancerous cells of the central nervous system from non-cancerous cells. Furthermore, it is an object of the present invention to provide a method, which allows to efficiently treat a tumor of the central nervous system including oligodendroglial tumors.

The first and the second object underlying the present invention are solved by a nucleic acid sequence derived from the genomic sequence encoding and/or regulating a Homo sapiens Cbp/p300-interacting transactivator, with Glu/Asp-rich carboxy-terminal domain 4 (CITED4), or an allelic variation thereof. In this context, a nucleic acid sequence encoding and/or regulating CITED4 corresponds to a segment of human chromosome 1 from position 40.995.843 to position 40.998.378, band 1 p34.2 (UCSC update from 2004-06-04), e.g. as depicted in SEQ ID NO: 39. Preferably, the inventive nucleic acid sequence is derived from a nucleic acid sequence according to SEQ ID NO: 1 (forming part of SEQ ID NO: 39), or an allelic variation thereof. More preferably, the inventive nucleic acid sequence is selected from one of the following sequences:
a) a nucleic acid sequence according to SEQ ID NO: 1, or an allelic variation thereof, having 10 to 200, 10 to 150, 10 to 100, 10 to 50, 10 to 40 or 10 to 30 nucleic acids in length;
b) a nucleic acid sequence complementary to a nucleic acid sequence according to SEQ ID NO: 1, or an allelic variation thereof, and having 10 to 200, 10 to150, 10 to 100, 10 to 50, 10 to 40 or 10 to 30 nucleic acids in length;
c) a nucleic acid sequence hybridizing under stringent conditions to a nucleic acid sequence according to SEQ ID NO: 1, or an allelic variation thereof, and having 10 to 200, 10 to150, 10 to 100, 10 to 50, 10 to 40 or 10 to 30 nucleic acids in length;
d) a nucleic acid sequence selected from any of SEQ ID NO's: 3 to 14;
e) a nucleic acid sequence sharing at least 80%, more preferably at least 85% and most preferably at least 90%, 95% or even 99% sequence identity with a nucleic acid sequence according to a), b), c) or d).

As defined above, the inventive nucleic acid sequence is derived from a nucleic acid sequence encoding CITED4, preferably according to SEQ ID NO: 1, or an allelic variation thereof. An inventive nucleic acid sequence may be DNA, cDNA, PNA, chromosomal DNA, extrachromosomal DNA, plasmid DNA, viral DNA, RNA and/or mRNA. Furthermore, an inventive nucleic acid sequence may be single stranded, double stranded, linear, circular, or a homo- or heteroduplex. Preparation and purification of such nucleic acids and/or derivatives may be carried out by a skilled person according to standard procedures (see e.g. Sambrook et al. 2001, Molecular Cloning: A Laboratory Manual, Cold Spring Harbor, NY).

An allelic variation of the nucleic acid sequence regulating and/or encoding CITED4 as defined above, typically comprises any nucleic acid sequence variants of a sequence regulating and/or encoding CITED4 as defined above due to (naturally occurring) alterations (mutations) of the native, i.e. non-altered, nucleic acid sequence according to SEQ ID NO's: 1 or 39, respectively. Those mutations include silent mutations, which do not result in changes of the encoded amino acid sequence and those in which mutations result in changes of the encoded amino acid sequence. More preferably, an allelic variation of the nucleic acid sequence according to SEQ ID NO: 1 or 39, respectively, comprises nucleic acid sequences sharing at least 90%, more preferably at least 95% and most preferably at least 99% sequence identity with a nucleic acid sequence according to SEQ ID NO: 1 or 39, respectively. Allelic variants of a nucleic acid sequence according to SEQ ID NO: 1 or 39, respectively, can be cloned by probing cDNA or genomic libraries from different individuals or tissues according to standard procedures.

In the present invention the term "identity" shall be understood as the degree of identity between two or more nucleic acid sequences, amino acid sequences, etc., due to deletions, substitutions or insertions of nucleic acids or amino acids, which may be determined by comparing these sequences using known methods such as computer based sequence alignments. For sequences without exact correspondence, a "% identity" of a first sequence may be determined with respect to a second sequence. In general, these two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or similar length, or over shorter, defined lengths (so-called local alignment), that is more suitable for sequences of unequal length. Methods for comparing the identity and homology of two or more sequences are well known in the art. Thus for instance, programs available in the Wisconsin Sequence Analysis Package, version 9.1 (Devereux et al., 1984, Nucleic Acids Res. 12, 387-395.), for example the programs BESTFIT and GAP, may be used to determine the % identity between two polynucleotides and the % identity and the % homology between two polypeptide sequences. BESTFIT uses the "local homology" algorithm (Smith and Waterman (1981), J. Mol. Biol. 147, 195-197) and finds the best single region of similarity between two sequences. Other programs for determining identity and/or similarity between sequences are also known in the art, for instance the BLAST family of programs (Altschul et al., 1990, J. Mol. Biol. 215, 403-410), accessible through the home page of the NCBI at world wide web site ncbi.nlm.nih.gov) and FASTA (Pearson (1990), Methods Enzymol. 183, 63-98; Pearson and Lipman (1988), Proc. Natl. Acad. Sci. U.S.A. 85, 2444-2448.).

The inventive nucleic acid sequence may comprise, as defined above, a nucleic acid sequence according to SEQ ID NO: 1, or an allelic variation thereof, wherein the inventive nucleic acid sequence has 10 to 200, 10 to 150, 10 to 100, 10 to 50, 10 to 40 or 10 to 30 nucleic acids in length. Preferably, these inventive nucleic acid sequences are selected from any section of the nucleic acid sequence according to SEQ ID NO: 1. More preferably, those nucleic acid sequences may be selected from at least one nucleic acid region at positions 1 to 100, 50 to 150, 100 to 200, 150 to 250, 200 to 300, 250 to 350, 300 to 400, 350 to 450, 400 to 500, or 450 to 552 of the nucleic acid sequence according to SEQ ID NO: 1.

Similarly, the inventive nucleic acid sequence may also comprise, as defined above, a nucleic acid sequence complementary to a nucleic acid sequence according to SEQ ID NO: 1, or an allelic variation thereof, and having 10 to 200, 10 to 150, 10 to 100, 10 to 50, 10 to 40 or 10 to 30 nucleic acids in length. Preferably, these nucleic acid sequences are selected from a complementary strand of any section of the nucleic acid sequence according to SEQ ID NO: 1. More preferably, those sections may be selected at least one nucleic acid region at positions 1 to 100, 50 to 150, 100 to 200, 150 to 250, 200 to 300, 250 to 350, 300 to 400, 350 to 450, 400 to 500, or 450 to 552 of the nucleic acid sequence according to SEQ ID NO: 1.

Additionally, as defined above, the inventive nucleic acid sequence may comprise a nucleic acid sequence hybridizing under stringent conditions to a nucleic acid sequence according to SEQ ID NO: 1, or an allelic variation thereof, and having 10 to 200, 10 to150, 10 to 100, 10 to 50, 10 to 40 or 10 to 30 nucleic acids in length. Preferably, those nucleic acid sequences hybridize under stringent conditions to one or more sections selected from any section of the nucleic acid sequence according to SEQ ID NO: 1. More preferably, those nucleic acid sequences hybridize under stringent conditions to at least one nucleic acid region at positions 1 to 100, 50 to 150, 100 to 200, 150 to 250, 200 to 300, 250 to 350, 300 to 400, 350 to 450, 400 to 500, or 450 to 552 of the nucleic acid sequence according to SEQ ID NO:1.

In the context of the present invention, the term "stringent conditions" preferably refers to hybridization conditions and to optional subsequent washing conditions, which those of ordinary skill in the art conventionally refer to as "stringent" (see Ausubel et al., Current Protocols in Molecular Biology, supra, Interscience, N.Y., 6.3 and 6.4 (1987, 1992), and Sambrook, J.C., Fritsch, E.F., and Maniatis, T. (2001) Molecular Cloning: A Laboratory Manual, Cold Spring Harbor Laboratory Press, Cold Spring Harbor, NY).

Stringent conditions, as defined herein, preferably refer to nucleic acid sequence(s) to be annealed. Therefore, hybridization temperatures of the nucleic acid sequence(s) to be annealed is(are) preferably selected to be about 5°C lower than the thermal melting point (Tₘ) for the specific nucleic acid sequence at a defined ionic strength and pH. In this context the Tₘ, represents the temperature (under defined ionic strength and pH) at which 50% of the target sequence hybridizes to a perfectly matched probe, i.e. a probe having a degree of complementarity of 100% with respect to the annealed sequence.

During annealing of inventive nucleic acid sequences, usually a pair of nucleic acid molecules, such as DNA-DNA, RNA-RNA and DNA-RNA, is formed, wherein such formation typically requires some degree of complementarity. Hybrids can tolerate mismatched base pairs in the double helix, but the stability of the hybrid is influenced by the degree of mismatch. The Tₘ of the mismatched hybrid decreases by 1°C for every 1-1.5% base pair mismatch. Varying the stringency of the hybridization conditions allows control over the degree of mismatch that will be present in the hybrid. The degree of stringency increases as the hybridization temperature increases and the ionic strength of the hybridization buffer decreases. Stringent hybridization conditions encompass temperatures of about 5-25°C below the Tₘ of the hybrid and a hybridization buffer having up to 1 M Na⁺. Higher degrees of stringency at lower temperatures can be achieved by addition of formamide, which reduces the Tₘ of the hybrid about 1 °C for each 1 % formamide in the buffer solution. Generally, such stringent conditions include temperatures of 20-70°C and a hybridization buffer containing up to 6 x SSC and 0-50% formamide. A higher degree of stringency can be achieved at temperatures from 40-70°C with a hybridization buffer having up to 4 x SSC and from 0-50% formamide. Highly stringent conditions typically encompass temperatures of 42-70°C with a hybridization buffer having up to 1 x SSC and 0-50% formamide. Different degrees of stringency can be used during hybridization and washing to achieve maximum specific binding to the target sequence. Typically, washes following hybridization are performed at increasing degrees of stringency to remove non-hybridized polynucleotide probes from hybridized complexes. The above conditions are intended to serve as a guidance and it is well within the abilities of a skilled person to adapt these conditions for use with a particular polynucleotide hybrid. As defined above, the Tₘ for a specific target sequence is the temperature (under defined conditions) at which 50% of the target sequence will hybridize to a perfectly matched probe sequence. Those conditions which influence the Tₘ include, the size and base pair content of the polynucleotide probe, the ionic strength of the hybridization solution, and the presence of destabilizing agents in the hybridization solution. Numerous equations for calculating Tₘ are known in the art, and are specific for DNA, RNA and DNA-RNA hybrids and polynucleotide probe sequences of varying length (see, for example, Sambrook et al., Molecular Cloning: A Laboratory Manual, Second Edition (Cold Spring Harbor Press 1989); Ausubel et al., (eds.), Current Protocols in Molecular Biology (John Wiley and Sons, Inc. 2001); Berger and Kimmel (eds.), Guide to Molecular Cloning Techniques, (Academic Press, Inc. 1987); and Wetmur, Crit. Rev. Biochem. Mol. Biol. 26: 227 (1990)). Sequence analysis software such as OLIGO 6.0 (LSR; Long Lake, MN) and Primer Premier 4.0 (Premier Biosoft International; Palo Alto, CA), as well as sites on the Internet, are available tools for analyzing a given sequence and calculating Tₘ based on user defined criteria. Such programs can also analyze a given sequence under defined conditions and identify suitable probe sequences. Typically, hybridization of longer polynucleotide sequences, >50 base pairs, is performed at temperatures of about 5-25°C, preferably about 5-10°C below the calculated Tₘ. For smaller probes, <50 base pairs, hybridization is typically carried out at the Tm or about 5-10°C below. This allows for the maximum rate of hybridization for DNA-DNA and DNA-RNA hybrids. The length of the polynucleotide sequence influences the rate and stability of hybrid formation. Smaller probe sequences, < 50 base pairs, reach equilibrium with complementary sequences rapidly, but may form less stable hybrids. Incubation times in the range of minutes to hours can be used to achieve hybrid formation. Longer probe sequences come to equilibrium more slowly, but form more stable complexes even at lower temperatures. Incubations are allowed to proceed overnight or longer. Generally, incubations are carried out for a period equal to three times the calculated Cot time. Cot time, the time it takes for the polynucleotide sequences to reassociate, can be calculated for a particular sequence by methods known in the art.

The base pair composition of inventive nucleic acid sequences will effect the thermal stability of the hybrid complex, thereby influencing the choice of hybridization temperature and the ionic strength of the hybridization buffer. A-T pairs are less stable than G-C pairs in aqueous solutions containing sodium chloride. Therefore, the higher the G-C content, the more stable the hybrid. Even distribution of G and C residues within the sequence also contributes positively to hybrid stability. In addition, the base pair composition can be manipulated to alter the Tₘ of a given sequence. For example, 5-methyldeoxycytidine can be substituted for deoxycytidine and 5-bromodeoxuridine can be substituted for thymidine to increase the Tₘ, whereas 7-deaza-2'-deoxyguanosine can be substituted for guanosine to reduce dependence on Tₘ. The ionic concentration of the hybridization buffer also affects the stability of the hybrid. Hybridization buffers generally may contain blocking agents such as Denhardt's solution (Sigma Chemical Co., St. Louis, Mo.), denatured salmon sperm DNA, tRNA, milk powders (BLOTTO), heparin or SDS, and a Na⁺ source, such as SSC (1x SSC: 0.15 M sodium chloride, 15 mM sodium citrate) or SSPE (1x SSPE: 1.8 M NaCl, 10 mM NaH₂PO₄, 1 mM EDTA, pH 7.7). By decreasing the ionic concentration of the buffer, the stability of the hybrid is normally decreased. Typically, hybridization buffers contain from between 10 mM - 1 M Na⁺. The addition of destabilizing or denaturing agents such as formamide, tetraalkylammonium salts, guanidinium cations or thiocyanate cations to the hybridization solution will alter the Tₘ of a hybrid. Typically, formamide may be used at a concentration of up to 50% to allow incubations to be carried out at more convenient and lower temperatures. Formamide also acts to reduce non-specific background when using RNA probes.

Following hybridization, the inventive nucleic acid sequences can be washed to remove non-hybridized nucleic acid molecules under stringent conditions, or under highly stringent conditions. Typical stringent washing conditions include washing in a solution of 0.5 x - 2 x SSC with 0.1% sodium dodecyl sulfate (SDS) at 55-65°C. Typical highly stringent washing conditions include washing in a solution of 0.1 x - 0.2 x SSC with 0.1% sodium dodecyl sulfate (SDS) at 50 - 65°C.

According to one preferred embodiment inventive nucleic acid sequences as defined above comprise or consist of nucleic acid sequences selected from any of SEQ ID NO's: 3 to 14 as defined in the following table:

**Table 1: Inventive nucleic acid sequences according to SEQ ID NO's: 3 to 14, wherein any sequence according SEQ ID NO's: 3-14 and may be used for expression analysis and sequences according to SEQ ID NO's: 3-12 further may be used for mutational analysis.**

| **SEQ ID NO:** | **Primer** | **Sequence** | **Amplicon lenght** |
|---|---|---|---|
| 3 | CITED4-P1-for | 5'-gggccaagacctagatgcag-3' | 229 bp |
| 4 | CITED4-P1-rev | 5'-aaaccaaacccgactggtg-3' | |
| 5 | CITED4-P2-for | 5'-cgcaaggtgcgcagtagt-3' | 229 bp |
| 6 | CITED4-P2-rev | 5'-ctctgcaccaggcggtag-3' | |
| 7 | CITED4-P3-for | 5'-catggccgaccacctgat-3' | 230 bp |
| 8 | CITED4-P3-rev | 5'-gaaagggctggaaggaggac-3' | |
| 9 | CITED4-P4-for | 5'-ctaccgcctggtgcagag-3' | 488 bp |
| 10 | CITED4-P4-rev | 5'-agtccgagaagcagtcgaac-3' | |
| 11 | CITED4-P5-for | 5'-catggacgccgaactcatc-3' | 212 bp |
| 12 | CITED4-P5-rev | 5'-agtcgggccctttctcctct-3' | |
| 13 | CITED4-P6-for | 5'-gggaggacagtttggcttca-3' | 109 bp |
| 14 | CITED4-P6-rev | 5'-gggagaggacacgatccaag-3' | |

An inventive nucleic acid may also comprise a nucleic acid sequence sharing at least 80%, more preferably at least 85% and most preferably at least 90%, 95% or even 99% sequence identity with a nucleic acid sequence according to a), b), c) or d). Such a nucleic acid sequence also includes nucleic acids, which may lead to an improved expression of their encoded protein in a selected host organism due to degeneration of their genetic code. Tables for appropriately adjusting a nucleic acid sequence are known to a skilled person. Preparation and purification of such nucleic acids and/or derivatives are usually carried out by standard procedures (see e.g. Sambrook et al., 2001, Molecular Cloning: A Laboratory Manual, Cold Spring Harbor, NY).

The inventive nucleic acid sequence(s) may be labelled for further detection, wherein a label is preferably selected from the group of labels comprising:
(i) radioactive labels, i.e. radioactive phosphorylation or a radioactive label selected from radioactive isotopes of sulphur, hydrogen, carbon, nitrogen, iod, etc.;
(ii) digoxygenin and digoxygenin-based detection systems;
(iii) fluorescent groups, wherein the fluorescent group may be selected from any fluorescent protein, e.g. from a group comprising fluorescein, the blue fluorescent protein (BFP), the green fluorescent protein (GFP), the photo activatable-GFP (PA-GFP), the yellow shifted green fluorescent protein (Yellow GFP), the yellow fluorescent protein (YFP), the enhanced yellow fluorescent protein (EYFP), the cyan fluorescent protein (CFP), the enhanced cyan fluorescent protein (ECFP), the monomeric red fluorescent protein (mRFP1), the kindling fluorescent protein (KFP1), aequorin, the autofluorescent proteins (AFPs), or the fluorescent proteins JRed, TurboGFP, PhiYFP and PhiYFP-m, tHc-Red (HcRed-Tandem), PS-CFP2 and KFP-Red (all available from EVRΩGEN, see also www.evrogen.com), or Alexa 350, Alexa 430, AMCA, BODIPY 630/650, BODIPY 650/665, BODIPY-FL, BODIPY-R6G, BODIPY-TMR, BODIPY-TRX, carboxyfluorescein, Cascade Blue, Cy3, Cy5, 6-FAM, Fluorescein, HEX, 6-JOE, Oregon Green 488, Oregon Green 500, Oregon Green 514, Pacific Blue, REG, Rhodamine Green, Rhodamine Red, ROX, TAMRA, TET, Tetramethylrhodamine, or Texas Red, or other suitable fluorescent proteins;
(iv) chemoluminescent groups, e.g. for time-resolved chemoluminescence, including lanthanoid complexes;
(v) metal colloids (e.g. gold, silver, etc) as particles;
(vi) groups for immobilization on a solid phase (e.g. biotin, streptag, antibodies, antigene, etc.); and
(vii) a combination of labels of two or more of the labels mentioned under (i) to (vi).

Preparation and purification of inventive nucleic acid sequences is typically carried out by standard procedures known to a skilled person (see e.g. Sambrook *et al.* 2001, Molecular Cloning: A Laboratory Manual, Cold Spring Harbor, NY).

According to alternative preferred embodiment, the inventive nucleic acid sequence is an (encoding and/or regulating) nucleic acid sequence derived from the complete nucleic acid sequence according to genomic sequence encoding a Homo sapiens Cbp/p300-interacting transactivator, with Glu/Asp-rich carboxy-terminal domain 4 (CITED4), as defined by SEQ ID NO: 39, an allelic variation thereof, or a part thereof. The sequence according to SEQ ID NO: 39 includes 5' upstream and 3' downstream sequences of the CITED4-gene as defined above, e.g. regulating sequences such as the promoter sequence of the CITED4-gene. More preferably, the inventive (encoding and/or regulating) nucleic acid sequence is selected from one of the following sequences:
a) a (regulating and/or encoding) nucleic acid sequence derived from a sequence according to SEQ ID NO: 39, or an allelic variation thereof, and having 10 to 200, 10 to150, 10 to 100, 10 to 50, 10 to 40 or 10 to 30 nucleic acids in length;
b) a (regulating and/or encoding) nucleic acid sequence complementary to a nucleic acid sequence derived from a sequence according to SEQ ID NO: 39, or an allelic variation thereof, and having 10 to 200, 10 to150, 10 to 100, 10 to 50, 10 to 40 or 10 to 30 nucleic acids in length;
c) a (regulating and/or encoding) nucleic acid sequence hybridizing under stringent conditions to a nucleic acid sequence derived from a sequence according to SEQ ID NO: 39, or an allelic variation thereof, and having 10 to 200, 10 to150, 10 to 100, 10 to 50, 10 to 40 or 10 to 30 nucleic acids in length;
d) a (regulating and/or encoding) nucleic acid sequence sharing at least 80%, more preferably at least 85% and most preferably at least 90%, 95% or even 99% sequence identity with a nucleic acid sequence according to a), b) or c).

The inventive (encoding and/or regulating) nucleic acid sequence may comprise, as defined above, a nucleic acid sequence derived from a sequence according to SEQ ID NO: 39, or an allelic variation thereof, wherein the inventive (encoding and/or regulating) nucleic acid sequence has 10 to 200, 10 to 150, 10 to 100, 10 to 50, 10 to 40 or 10 to 30 nucleic acids in length. Preferably, these inventive nucleic acid sequences are selected from any section of the nucleic acid sequence according to SEQ ID NO: 39. More preferably, those nucleic acid sequences may be selected from at least one nucleic acid region at positions 1 to 100, 50 to 150, 100 to 200, 150 to 250, 200 to 300, 250 to 350, 300 to 400, 350 to 450, 400 to 500, 450 to 550, 500 to 600, 550 to 650, 600 to 700, 650 to 750, 700 to 800, 750 to 850, 800 to 900, 850 to 950, 900 to 1000, 950 to 1050, 1000 to 1100, 1050 to 1150, 1100 to 1200, 1150 to 1250, 1200 to 1300, 1250 to 1350, 1300 to 1400, 1350 to 1450, 1400 to 1500, 1450 to 1550, 1500 to 1600, 1550 to 1650, 1600 to 1700, 1650 to 1750, 1700 to 1800, 1750 to 1850, 1800 to 1900, 1850 to 1950, 1900 to 2000, 1950 to 2050, 2000 to 2100, 2050 to 2150, 2100 to 2200, 2150 to 2250, 2200 to 2300, 2250 to 2350, 2300 to 2400, 2350 to 2450, 2400 to 2500 or 2450 to 2536 of the nucleic acid sequence according to SEQ ID NO: 39.

Similarly, the inventive (encoding and/or regulating) nucleic acid sequence may also comprise, as defined above, a nucleic acid sequence complementary to a nucleic acid sequence derived from a sequence according to SEQ ID NO: 39, or an allelic variation thereof, and having 10 to 200, 10 to150, 10 to 100, 10 to 50, 10 to 40 or 10 to 30 nucleic acids in length. Preferably, these nucleic acid sequences are selected from a complementary strand of any section of the nucleic acid sequence according to SEQ ID NO: 39. More preferably, those sections may be selected at least one nucleic acid region at positions 1 to 100, 50 to 150, 100 to 200, 150 to 250, 200 to 300, 250 to 350, 300 to 400, 350 to 450, 400 to 500, 450 to 550, 500 to 600, 550 to 650, 600 to 700, 650 to 750, 700 to 800, 750 to 850, 800 to 900, 850 to 950, 900 to 1000, 950 to 1050, 1000 to 1100, 1050 to 1150, 1100 to 1200, 1150 to 1250, 1200 to 1300, 1250 to 1350, 1300 to 1400, 1350 to 1450, 1400 to 1500, 1450 to 1550, 1500 to 1600, 1550 to 1650, 1600 to 1700, 1650 to 1750, 1700 to 1800, 1750 to 1850, 1800 to 1900, 1850 to 1950, 1900 to 2000, 1950 to 2050, 2000 to 2100, 2050 to 2150, 2100 to 2200, 2150 to 2250, 2200 to 2300, 2250 to 2350, 2300 to 2400, 2350 to 2450, 2400 to 2500 or 2450 to 2536 of the nucleic acid sequence according to SEQ ID NO: 39.

Additionally, as defined above, the inventive (encoding and/or regulating) nucleic acid sequence may comprise a nucleic acid sequence hybridizing under stringent conditions to a nucleic acid sequence derived from a sequence according to SEQ ID NO: 39, or an allelic variation thereof, and having 10 to 200, 10 to 150, 10 to 100, 10 to 50, 10 to 40 or 10 to 30 nucleic acids in length. Preferably, those nucleic acid sequences hybridize under stringent conditions to one or more sections selected from any section of the nucleic acid sequence according to SEQ ID NO: 39. More preferably, those nucleic acid sequences hybridize under stringent conditions to at least one nucleic acid region at positions 1 to 100, 50 to 150, 100 to 200, 150 to 250, 200 to 300, 250 to 350, 300 to 400, 350 to 450, 400 to 500, 450 to 550, 500 to 600, 550 to 650, 600 to 700, 650 to 750, 700 to 800, 750 to 850, 800 to 900, 850 to 950, 900 to 1000, 950 to 1050, 1000 to 1100, 1050 to 1150, 1100 to 1200, 1150 to 1250, 1200 to 1300, 1250 to 1350, 1300 to 1400, 1350 to 1450, 1400 to 1500, 1450 to 1550, 1500 to 1600, 1550 to 1650, 1600 to 1700, 1650 to 1750, 1700 to 1800, 1750 to 1850, 1800 to 1900, 1850 to 1950, 1900 to 2000, 1950 to 2050, 2000 to 2100, 2050 to 2150, 2100 to 2200, 2150 to 2250, 2200 to 2300, 2250 to 2350, 2300 to 2400, 2350 to 2450, 2400 to 2500 or 2450 to 2536 of the nucleic acid sequence according to SEQ ID NO: 39.

According to a preferred embodiment, an inventive (regulating and/or encoding) nucleic acid sequence comprises or consists of the following sequences:

**Table 2: Inventive (regulating and/or encoding) nucleic acid sequences according to SEQ ID NO: 15 to 34,**

| **SEQ ID NO:** | **Primer** | **Sequence** | **Amplicon lenght** |
|---|---|---|---|
| 15 | CITED4-P7-for | 5'-tttggttagtttaagttttattttagt-3' | 330 bp |
| 16 | CITED4-P7-rev | 5'-aatcacaaaaacccgcaacctataa-3' | |
| 17 | CITED4-P8-for | 5'-ttataggttgcgggtttttgtgatt-3' | 268 bp |
| 18 | CITED4-P8-rev | 5'-acctctacaccaaacgataacc-3' | |
| 19 | CITED4-Met-F1 | 5'-gcggccgcttttttgggatagaatttaaatagttt-3' | F1/R1 296 bp |
| 20 | CITED4-Met-R1 | 5'-ccaaactaaactacaataacgcta-3' | |
| 21 | CITED4-Met-F2 | 5'-gcggccgctttggttagtttaagttttattttagt-3' | F2/R2 333bp |
| 22 | CITED4-Met-R2 | 5'-aatcacaaaaacccgcaacctataa-3' | |
| 23 | CITED4-Met-F3 | 5'-gcggccgcttataggttgcgggtttttgtgatt-3' | F3/R3 276 bp |
| 24 | CITED4-Met-R3 | 5'-acctctacaccaaacgataacc-3' | |
| 25 | CITED4MSP-metF | 5'-cgcggttcgtttagaagcgtttc-3' | CITED4MSP-metF/ R1:102 bp |
| 26 | CITED4MSP-metR1 | 5'-cgcgtcgcgtaactaaactcg-3' | |
| 27 | CITED4MSP-metR2 | 5'-cgaaacctccgcgtcgcg-3' | CITED4MSP-metF/ R2: 111 bp |
| 28 | CITED4MSP-F | 5'-tgtggtttgtttagaagtgttttgtttagttaatg-3' | CITED4MSP-F/ R1:102 bp |
| 29 | CITED4MSP-R1 | 5'-cacatcacataactaaactcaacatattcttata-3' | |
| 30 | CITED4MSP-R2 | 5'-caaaacctccacatcacataactaaactca-3' | CITED4MSP-metF/ R2: 111 bp |
| 31 | CITED4BisF2gegen | 5'-gcggccgcggttataaaggttcgtaatttgtag-3' | CITED4BisF2gegen/ R2gegen333bp |
| 32 | CITED4BisR2gegen | 5'-tctaatcaattcaaactctaccccaac-3' | |
| 33 | CITED4-Met-F4 | 5'-gcggccgcGGGTTAAGATTTAGATGTAGG-3' | CITED4-Met-F4/ R2: 174 bp |
| 34 | CITED4BisR4gegen | 5'-aaaacaaaaccaaaacctaaatacaaa-3' | CITED4BisF2/ bp R4gegen:180 bp |

Similarly, inventive (regulating and/or encoding) nucleic acid sequences may be labelled. With respect to labels, hybridization and sequence identity it is referred to the definitions as set forth above. Also, any definition with respect to lengths of inventive (regulating and/or encoding) nucleic acid sequences applies as disclosed above for inventive nucleic acid sequences.

According to a preferred embodiment, an inventive nucleic acid molecule is a double stranded nucleic acid, preferably a dsRNA. Such dsRNAs may be used e.g. for RNA interfering methods (RNAi). "RNA interference" or "RNAi" is a term initially applied to a phenomenon observed in plants and worms where double-stranded RNA (dsRNA) blocks gene expression in a specific and post-transcriptional manner. Without being bound by theory, RNAi appears to involve mRNA degradation; however, the biochemical mechanisms are currently an active area of research. Despite some uncertainty regarding the mechanism of action, RNAi provides a useful method of inhibiting gene expression *in vitro* or *in vivo.* dsRNAs according to the present invention may be occur as (i) siRNA, (ii) long ds RNA, containing one or more identical or different siRNA(s) within its long dsRNA-sequence, or (iii) siRNA based hairpin-loop-RNA or (iv) miRNA-based hairpin-loop-siRNA. In the context of the present invention any of the afore mentioned alternatives are considered to be "dsRNA". With respect to (i) an siRNA is typically synthesized and incorporated intracellularly by avoiding the dicing-step in the RISC-complex, which typically leads to a sequence specific mRNA-degradation (of the target sequence). A long dsRNA according to (ii) is typically a precursor form of siRNA (according to (i)), which is processed by a dicing-step into mature siRNA.

As used herein, the term siRNA is meant to be equivalent to other terms used to describe nucleic acid molecules that are capable of mediating sequence specific RNAi, for example double-stranded RNA (dsRMA), micro-RNA (miRNA), short hairpin RNA (shRNA), chemically-modified siRNA, post-transcriptional gene silencing RNA (ptgsRNA), translational silencing RNA, and other RNA molecules. Long double stranded interfering RNAs, such a miRNAs, appear to tolerate mismatches more readily than do short double stranded RNAs. In addition, as used herein, the term RNAi is meant to be equivalent to other terms used to describe sequence specific RNA interference, such as posttranscriptional gene silencing, or an epigenetic phenomenon. For example, siRNA molecules of the invention can be used to epigenetically silence a CITED4 gene at both the post-transcriptional level or the pre-transcriptional level. In a non-limiting example, epigenetic regulation of gene expression by siRNA molecules of the invention can result from siRNA mediated modification of chromatin structure and thereby alter gene expression (see, for example, Allshire (2002) Science 297,1818-1819 ; Volpe et al. (2002) Science 297, 1833-1837; Jenuwein (2002) Science 297, 2215-2218 ; and Hall et al. (2002) Science 297, 2232-2237).

An siRNA can be designed to target a partial or the complete region of the coding or non-coding sequence of the CITED4 gene as defined above, or an mRNA thereof (e.g. as defined bey either SEQ ID NO: 1 or 39). (These sequences are herein also defined as "target" or "target sequence"). An inventive siRNA is typically a double-stranded polynucleotide molecule comprising self-complementary sense and antisense regions, wherein the antisense region comprises nucleotide sequence that is complementary to nucleotide sequence in a target nucleic acid molecule or a portion thereof and the sense region has a nucleotide sequence corresponding to the target nucleic acid sequence or a portion thereof. The siRNA can be assembled from two separate oligonucleotides, where one strand is the sense strand and the other is the antisense strand and wherein the antisense and sense strands are self-complementary. The siRNA can be assembled from a single oligonucleotide, where the self-complementary sense and antisense regions of the siRNA are linked by means of nucleic acid-based or non-nucleic acid-based linker(s). The siRNA can be a polynucleotide with a hairpin secondary structure, having self-complementary sense and antisense regions. The siRNA can-be a circular single-stranded polynucleotide having two or more loop structures and a stem comprising self-complementary sense and antisense regions, wherein the circular polynucleotide can be processed either *in vivo* or *in vitro* to generate an active siRNA molecule capable of mediating RNAi. The siRNA can also comprise a single stranded polynucleotide having nucleotide sequence complementary to nucleotide sequence in a target nucleic acid molecule or a portion thereof, wherein the single stranded polynucleotide can further comprise a terminal phosphate group, such as a 5'-phosphate (see for example Martinez et al. (2002) Cell 110, 563- 574 and Schwarz et al. (2002) Molecular Cell 10, 537-568), or 5', 3'-diphosphate.

Typically, an inventive siRNA comprises any length that is effective for inhibiting expression of the CITED4 gene as defined above. Preferably, siRNA molecules comprise a length of 6 to 50 nucleotides. More preferably, the length of an effective siRNA comprises 17 nucleotides to 25 nucleotides in length, even more preferably 19 to 25 nucleotides and most preferably 21 to 23 nucleotides in length.

In certain embodiments, the siRNA molecule of the invention comprises separate sense and antisense sequences or regions, wherein the sense and antisense regions are covalently linked by nucleotide or non-nucleotide linker molecules as is known in the art, or are alternately non-covalently linked by ionic interactions, hydrogen bonding, Van der Waal's interactions, hydrophobic interactions, and/or stacking interactions.

As used herein, siRNA molecules need not be limited to those molecules containing only RNA, but further encompass chemically-modified nucleotides, non-nucleotides and/or labels as defined above. siRNA molecules may comprise no or at least about 5, 10, 20, 30, 40, 50, 60, 70, 80, 90, 95 or 100% ribonucleotides. siRNA molecules that do not require the presence of ribonucleotides within the siRNA molecule to support RNAi may however have an attached linker or linkers or other attached or associated groups, moieties, or chains containing one or more nucleotides with 2'-OH groups.

Preferably, a molecule mediating RNAi has a 2 nucleotide 3'overhang. If the RNAi molecule is expressed in a cell from a construct, for example from a hairpin molecule or from an inverted repeat, then the endogenous cellular machinery will create the overhangs. Considerations to be taken into account when designing an RNAi molecule include, e.g. the sequence to be targeted, secondary structure of the RNA target and binding of RNA binding proteins. Methods of optimizing siRNA sequences will be evident to the skilled worker. Typical algorithms and methods are described in Vickers et al. (2003) J Biol Chem 278: 7108-7118; Yang et al. (2003) Proc Natl Acad Sci USA 99 : 9942-9947; Far et al. (2003) Nuc. Acids Res. 31: 4417-4424; and Reynolds et al. (2004) Nature Biotechnology 22: 326-330. Methods of preparing inventive siRNAs are conventional and known to a skilled person. *ln vitro* methods include processing the polyribonucleotide sequence in a cell-free system (e.g. digesting long dsRNAs with RNAse III or Dicer), transcribing recombinant double stranded DNA *in vitro.* and, preferably, chemical synthesis of nucleotide sequences. See, e.g. Tuschl et al. (1999) Genes & Dev. 13 : 3191-3197. *In vivo* methods include (1) transfecting DNA vectors into a cell such that a substrate is converted into siRNA *in vivo* (see, for example, Kawasaki et al. (2003) Nucleic Acids Res 31: 700-707; Miyagishi et al. (2003) Nature Biotechnol 20: 497-500; Lee et al. (2002) Nature Biotechnol 20:500-505 ; Brummelkamp et al. (2002) Science 296 : 550-553; McManus et al. (2002) RNA 8 : 842-850; Paddison et al. (2002) Gene Dev 16 : 948-958; Paddison et al. (2002) Proc Natl Acad Sci USA 99 : 1443-1448); Paul et al. (2002) Nature Biotechnol 20: 505-508; Sui et al. (2002) Proc Natl Acad Sci USA 99 : 5515-5520; Yu et al. (2002) Proc Natl Acad Sci USA 99: 6047-6052) (2) expressing short hairpin RNAs from plasmid systems using RNA polymerase (polIII) promoters (see, for example, Kawasaki *et al.,* supra; Miyagishi *et al*., supra; Lee *et al.,* supra; Brummelkamp *et al.,* supra; McManus *et al*., supra), Paddison *et al*., supra (both); Paul *et al.,* supra, Sui *et al*., supra ; and Yu *et al.,* supra) and/or (3) expressing short RNA from tandem promoters (see, for example, Miyagishi *et al.,* supra; Lee *et al.,* supra). When synthesized *in vitro,* a typical µM scale RNA synthesis provides about 1 mg of siRNA, which is sufficient for about 1000 transfection experiments using a 24-well tissue culture plate format.

For reviews and more general description of inhibitory RNAs, see Lau et al. (2003 Aug) Sci Amer pp 34-41; McManus et al. (2002) Nature Rev Genetics 3,737-747 ; and Dylxhoorn et al. (2003) Nature Rev Mol Cell Bio 4: 457-467. For further guidance regarding methods of designing and preparing siRNAs, testing them for efficacy, and using them in methods of RNA interference (both *in vitro* and *in vivo*), see, e.g. Allshire (2002) Science 297 : 1818-1819; Volpe et al. (2002) Science 297: 1833-1837; Jenuwein (2002) Science 297 : 2215-2218; Hall et al. (2002) Science 297 2232-2237; Hutvagner et al. (2002) Science 297: 2056-60; McManus et al. (2002) RNA 8 : 842-850; Reinhart et al. (2002) Genes Dev. 16: 1616-1626; Reinhart et al. (2002) Science 297 :1831; Fire et al. (1998) Nature 391: 806-811: Moss (2001) Curr Biol II : S772-5; Brummelkamp et al. (2002) Science 296: 550-553; Bass (2001) Nature 411 428-429; and Elbashir et al. (2001) Nature 411: 494-498; US Pat. 6,506,559; Published US Pat App. 20030206887; and PCT applications WO 99/07409, WO 99/32619, WO 00/01846, WO 00/44914, WO 00/44895, WO 01/29058, WO 01/36646, WO 01/75164, WO 01/92513, WO 01/29058, WO 01/89304, WO 01/90401, WO 02/16620, and WO 02/29858. Ribozymes and siRNAs can take any of the forms, including modified versions, described for antisense nucleic acid molecules; and they can be introduced into cells as oligonucleotides (single or double stranded), or in an expression vector.

The present invention is also directed to amino acid sequences encoded by any of the inventive nucleic acid sequences as defined above. Preferably, such amino acid sequences are amino acid sequences derived from Homo sapiens Cbp/p300-interacting transactivator, with Glu/Asp-rich carboxy-terminal domain 4 (CITED4) as defined above. More preferably, the inventive amino acid sequences are derived from a complete or partial amino acid sequence according to SEQ ID NO: 2 (amino acid sequence of CITED4) or are encoded by a complete or partial nucleic acid sequence according to SEQ ID NO: 1. Even more preferably, the inventive amino acid sequence is selected from one of the following sequences:
a) an amino acid sequence according to SEQ ID NO: 2, having 10 to 175, 10 to 150, 10 to 100, 10 to 50, 10 to 25 or 10 to 15 or 20 to 175, 20 to 150, 20 to 100, 20 to 50, or 20 to 25 amino acids in length;
b) an amino acid sequence, sharing at least 80%, more preferably at least 85% and most preferably at least 90%, 95% or even 99% sequence identity with an amino acid sequence according to a);
c) an amino acid sequence, sharing 70 to 80%, more preferably up 75 to 85% and most preferably up to 80 to 90%, 90 to 95% or even 95 to 99% sequence identity with an amino acid sequence according SEQ ID NO: 2.

With respect to the definition of "identity" it is referred to the definitions given above.

As referred to above, the present invention may provide an amino acid sequence according to SEQ ID NO: 2, having 10 to 175, 10 to 150, 10 to 100, 10 to 50, 10 to 25 or 10 to 15 amino acids in length. Alternatively, the inventive amino acid sequence according to SEQ ID NO: 2 may have 20 to 175, 20 to 150, 20 to 100, 20 to 50, or 20 to 25 amino acids in length. Preferably, such an inventive amino acid sequence comprises an epitope-bearing portion of a CITED4 polypeptide, e.g. as defined by SEQ ID NO: 2. Such an epitope-bearing portion may comprise an "immunogenic epitope", which - in the context of the present invention - represents a part of a protein that elicits an antibody response when the entire inventive amino acid is used as an immunogen. Immunogenic epitope-bearing peptides can be identified using standard methods (see, for example, Geysen et al., Proc. Nat'l Acad. Sci. USA 81: 3998 (1983)). Alternatively, inventive amino acid sequences may comprise an "antigenic epitope," which represents a region of a protein molecule to which an antibody, preferably an inventive antibody, can specifically bind. Certain epitopes consist of a linear or contiguous stretch of amino acids, and the antigenicity of such an epitope is not disrupted by denaturing agents. It is known in the art that relatively short synthetic peptides that can mimic epitopes of a protein can be used to stimulate the production of antibodies against the protein (see, for example, Sutcliffe et al., Science 219: 660 (1983)). Accordingly, inventive antigenic epitope bearing amino acid sequences are useful to raise antibodies that bind with the inventive amino acids described herein or with a CITED4 polypeptide according to SEQ ID NO: 2. Inventive antigenic epitope-bearing amino acid sequences preferably contain at least five to ten amino acids, at least ten to fifteen amino acids, or about 15 to about 30 amino acids of a sequence according to SEQ ID NO: 2. Such inventive epitope-bearing amino acid sequences can be produced by fragmenting a CITED4 polypeptide as defined above (e.g. according to SEQ ID NO: 2), or by chemical peptide synthesis. Moreover, epitopes can be selected by phage display of random peptide libraries (see, for example, Lane and Stephen, Curr. Opin. Immunol. 5: 268 (1993), and Cortese et al., Curr. Opin. Biotechnol. 7: 616 (1996)). Standard methods for identifying epitopes (and producing antibodies) from small peptides that comprise an epitope are described, for example, by Mole, "Epitope Mapping," in Methods in Molecular Biology, Vol. 10, Manson (ed.), pages 105-116 (The Humana Press, Inc. 1992), Price, "Production and Characterization of Synthetic Peptide-Derived Antibodies, "in Monoclonal Antibodies : Production, Engineering, and Clinical Application, Ritter and Ladyman (eds.), pages 60-84 (Cambridge University Press 1995), and by Coligan et al. (eds.), Current Protocols in Immunology, pages 9.3.1-9.3.5 and sections 9.4.1-9.4.11 (John Wiley & Sons 1997).

Another embodiment of the present invention refers to vectors, in particular to expression vectors, containing at least one inventive nucleic acid sequence or one inventive (regulating and/or encoding) nucleic acid sequence as defined above. A "vector" within the meaning of the present invention advantageously comprises at least one inventive nucleic acid sequence or one inventive (regulating and/or encoding) nucleic acid sequence and, if necessary, additional elements suitable for directing expression of the encoded inventive amino acid sequences as defined above. One class of vectors as used herein utilizes DNA elements that provide autonomously replicating extrachromosomal plasmids derived from animal viruses (e.g. bovine papilloma virus, polyomavirus, adenovirus, or SV40, etc.). A second class of inventive vectors as used herein relies upon the integration of the desired gene sequences into the host cell chromosome.

Inventive vectors, as defined above, are typically prepared by inserting at least one inventive nucleic acid sequence or one inventive (regulating and/or encoding) nucleic acid sequence into suitable vectors. Such suitable vectors are known to a skilled person and may be reviewed e.g. in "Cloning Vectors" (Eds. Pouwels P. H. et al. Elsevier, Amsterdam-New York-Oxford, 1985, ISBN 0 444 904018). Suitable vectors are also intended to include any vector known to a skilled person, such as plasmids, phages, viruses such as SV40, CMV, Baculo virus, Adeno virus, Sindbis virus, transposons, IS-elements, phasmids, phagemides, cosmides, linear or circular DNA. Such vectors can be replicated autonomously in a host organism or replicated chromosomally. For integration in mammalian cells linear DNA is typically used. Preferably, the vector type used for the present invention corresponds to the specific host cell requirements. Suitable commercially available expression vectors, into which the inventive nucleic acids may be inserted, include pSPORT, pBluescriptilSK, the baculovirus expression vector pBlueBac, and the prokaryotic expression vector pcDNAll, all of which may be obtained from Invitrogen Corp., San Diego, CA.

An inventive vector typically combines the inventive nucleic acid sequences or inventive (regulating and/or encoding) nucleic acid sequences with other elements, which e.g. control expression of the encoded (inventive) amino acid sequences. Such additional elements are preferably selected from regulation sequences, origins of replication (if the vectors are replicated autonomously) and marker genes. Regulation sequences in the scope of the present invention are any elements known to a skilled person having an impact on expression on transcription and/or translation of inventive nucleic acid sequences or inventive (regulating and/or encoding) nucleic acid sequences. Regulation sequences include, apart from promoter sequences so-called enhancer sequences, which may lead to an increased expression due to enhanced interaction between RNA polymerase and DNA. Further regulation sequences of inventive vectors are transcriptional regulatory and translational initiation signals, so-called, "terminator sequences", "stability leader sequences", etc. or partial sequences thereof.

Generally, all naturally occurring promoters may be used in an expression vector according to the present invention. Such promoters may be selected from any eukaryotic, prokaryotic, viral, bacterial, plant, human or animal, e.g. mammalian promoters. Suitable promoters include, for example, the cytomegalovirus promoter, the lacZ promoter, the gal 10 promoter and the AcMNPV polyhedral promoter, promoters such as cos-, tac-, trp-, tet-, trp-tet-, lpp-, lac-, lpp-lac-, laclq-, T7-, T5-, T3-, gal-, trc-, ara-, SV40-, SP6, l-PR- or the l-PL-promoter, advantageously being found in gram-negative bacteria. Additionally, promoters may be obtained from gram-positive promoters such as amy and SPO2, yeast promoters, such as ADC1, MFa, AC, P-60, CYC1, GAPDH or mammalian promoters such as CaM-Kinasell, CMV, Nestin, L7, BDNF, NF, MBP, NSE, beta-globin, GFAP, GAP43, tyrosine hydroxylase, Kainat-receptor-subunit 1, glutamate-receptor-subunit B. Finally, synthetic promoters may be used advantageously. Promoter sequences, as contained in an inventive vector, may also be inducible, to allow modulation of expression (e.g. by the presence or absence of nutrients or other inducers in the growth medium). One example is the lac operon obtained from bacteriophage lambda plac5, which can be induced by IPTG. Finally, a promoter as defined above may be linked with an inventive nucleic acid sequence such that the promoter is positioned 5' "upstream" of the inventive nucleic acid sequence or inventive (regulating and/or encoding) nucleic acid sequence.

Enhancer sequences for upregulating expression of inventive nucleic acid sequences or inventive (regulating and/or encoding) nucleic acid sequences are preferably another constituent of an inventive vector as defined above. Such enhancer sequences are typically located in the non-coding 3' region of the vector. Enhancer sequences as employed herewith may be obtained from any eukaryotic, prokaryotic, viral, bacterial, plant, human or animal, e.g. mammalian hosts, preferably in association with the corresponding promoters as defined above.

Furthermore, an inventive vector may contain transcriptional and/or translational signals, preferably transcriptional and/or translational signals recognized by an appropriate host, such as transcriptional regulatory and translational initiation signals. Transcriptional and/or translational signals may be obtained from any eukaryotic, prokaryotic, viral, bacterial, plant, human or animal, e.g. mammalian hosts, preferably in association with the corresponding promoters as defined above. A wide variety of transcriptional and translational regulatory sequences may be employed herein, depending upon the nature of the host. To the extent that the host cells recognizes the transcriptional regulatory and translational initiation signals associated with an inventive nucleic acid, the 5' region adjacent to the inventive nucleic acid sequence or inventive (regulating and/or encoding) nucleic acid sequence may be retained and employed for transcriptional and translational regulation in an inventive vector. This region typically will include those sequences involved with initiation of transcription and translation, such as the TATA box, capping sequence, CAAT sequence, and the like. Typically, this region will be at least about 150 base pairs long, more typically about 200 bp, and rarely exceeding about 1 to 2 kb.

Transcriptional initiation regulatory signals may be selected that allow to control repression or activation such that expression of the genes can be modulated. One such controllable modulation technique is the use of regulatory signals that are temperature-sensitive in order to repress or initiate expression by changing the temperature. Another controllable modulation technique is the use of regulatory signals that are sensitive to certain chemicals. Transcription and/or translational signals also include transcriptional termination regulatory sequences, such as a stop signal and a polyadenylated region. Preferably, transcriptional termination regulatory sequences are located in the non-coding 3' region of an inventive vector containing the inventive nucleic acid sequence or inventive (regulating and/or encoding) nucleic acid sequence. Suitable termination sequences include, for example, the bovine growth hormone, SV40, lacZ and AcMNPV polyhedral polyadenylation signals.

The inventive expression vectors may also include other sequences for optimal expression of inventive amino acid sequences or inventive (regulating and/or encoding) nucleic acid sequence. Such sequences include stability leader sequences, which provide for stability of the expression product; secretory leader sequences, which provide for secretion of the expression product; and restriction enzyme recognition sequences, which provide sites for cleavage by restriction endonucleases. All of these materials are known in the art and are commercially available (see, for example, Okayama (1983), Mol. Cell. Biol., 3: 280).

Additionally to regulation sequences as defined above, an autonomously replicating inventive vector typically comprises an origin of replication. Suitable origins of replication include, for example, ColE1, pSC101, SV40 and M13 origins of replication.

Finally, marker genes may also be contained in an inventive vector. Such marker genes preferably allow phenotypic selection of transformed host cells. A marker gene may provide prototrophy to an auxotrophic host, biocide resistance (e.g. antibiotic resistance genes) and the like. The selectable marker gene may either be directly linked to the inventive nucleic acid sequences or inventive (regulating and/or encoding) nucleic acid sequences to be expressed, or may be introduced into the same cell by co-transfection. Examples of selectable markers include neomycin, ampicillin, hygromycin, kanamycin, kanamycin-neomycin and the like.

Another embodiment of the present invention refers to host cells, being transformed with at least one inventive vector as defined above. Advantageously, an inventive host cell which is used as an expression system according to the present invention may be formed by combining a host cell and at least one inventive expression vector. Such host cells are encompassed by the present invention as well. Suitable host cells are to be understood herein as any cell, allowing expression of inventive nucleic acid sequences or inventive (regulating and/or encoding) nucleic acid sequences or of such sequences in combination with additional sequences, particularly with regulation sequences. Any cultivated eukaryotic (yeast, mammalian cells), prokaryotic, viral, bacterial, plant, human and animal cell line may be used as a host cell, or cell lines being impaired by infections, e.g. HIV, and/or cell lines of the immune system or of the central nervous system. Preferably, cells from multi-cellular organisms are selected as host cells for expression of inventive nucleic acid sequences or inventive (regulating and/or encoding) nucleic acid sequences. Cells from multi-cellular organisms are particularly preferred, if post-translational modifications, e.g. glycosylation of the encoded proteins, are required (N and/or O coupled). In contrast to prokaryotic cells, higher eukaryotic cells may permit these modifications to occur. The skilled person is aware of a plurality of established cell lines suitable for this purpose, e.g. 293T (embryonic kidney cell line), HeLa (human cervix carcinoma cells) and further cell lines, including cell lines established for laboratory use, such as HEK293-, Sf9- or COS-cells. Particularly preferred are human cells, more preferably cells of the central nervous system, e.g. brain cells. Host cells may also be bacteria, such as Escherichia coli, eukaryotic microorganisms, such as Saccharomyces cerevisiae (Stinchcomb et al., Nature, 282:39, (1997)), etc..

Antibodies, particular anti-CITED4-antibodies are also encompassed by the present invention. Antibodies according to the present invention are preferably polyclonal or monoclonal antibodies to a CITED4-polypeptide as well as anti-idiotype antibodies, and specifically bind to a CITED4-polypeptide according to SEQ ID NO: 2, to inventive amino acid sequences or to nucleic acid sequences according to the present invention including inventive (regulating and/or encoding) nucleic acid sequences. Also encompassed by the present invention are antibody-fragments of such anti-CITED4 antibodies. In general, inventive anti-CITED4 antibodies may be produced using inventive nucleic acid sequences, inventive (regulating and/or encoding) nucleic acid sequences or inventive amino acid sequences, preferably including antigenic CITED4 epitope-bearing amino acid sequences. As defined above, antigenic epitope-bearing amino acid sequences of the present invention typically contain a sequence of at least five to ten amino acids, at least ten to fifteen amino acids, or about 15 to about 30 amino acids contained within SEQ ID NO: 2. However, peptides or polypeptides comprising a larger portion of an amino acid sequence of the invention, containing from 30 to 50 amino acids, or any length up to and including the entire amino acid sequence of a polypeptide according to the invention or the entire sequence of CITED4 according to SEQ ID NO: 2, are also useful for inducing antibodies that bind with CITED4. It is furthermore desirable that the inventive epitope-bearing amino acid sequence is selected to provide substantial solubility in aqueous solvents (i. e., the sequence includes relatively hydrophilic residues, while hydrophobic residues are preferably avoided). Moreover, amino acid sequences containing proline residues may also be desirable for antibody production.

According to the present invention, anti-CITED4-antibodies may be polyclonal antibodies. Polyclonal antibodies may be prepared using methods well-known to those of skill in the art e.g. using inventive nucleic acid sequences, inventive (regulating and/or encoding) nucleic acid sequences, inventive amino acid sequences or a CITED4-polypeptide according to SEQ ID NO: 2 (see, for example, Green et al., "Production of Polyclonal Antisera," in Immunochemical Protocols (Manson, ed.), pages 1-5 (Humana Press 1992), and Williams et al., "Expression of foreign proteins in E. coli using plasmid vectors and purification of specific polyclonal antibodies" in DNA Cloning 2: Expression Systems, 2nd Edition, Glover et al. (eds.), page 15 (Oxford University Press 1995). When preparing polyclonal antibodies, the immunogenicity of inventive nucleic acid sequences, inventive (regulating and/or encoding) nucleic acid sequences, inventive amino acid sequences or a CITED4-polypeptide according to SEQ ID NO: 2 may be increased by using of an adjuvant, such as alum (aluminum hydroxide) or Freund's complete or incomplete adjuvant. If the inventive amino acid sequence or the CITED4 polypeptide according to SEQ ID NO: 2 used as an immunogen is "hapten-like", such amino acid sequence or polypeptide may be advantageously joined or linked to a macromolecular carrier (such as keyhole limpet hemocyanin (KLH), bovine serum albumin (BSA) or tetanus toxoid) for immunization. Although polyclonal antibodies are typically raised in animals such as horses, cows, dogs, chicken, rats, mice, rabbits, guinea pigs, goats, or sheep, an anti-CITED4-antibody of the present invention may also be derived from a subhuman primate antibody. General techniques for raising diagnostically and therapeutically useful antibodies in baboons may be found, for example, in Goldenberg et al., International Patent Publication No. WO 91/11465, and in Losman et al., Int. J. Cancer 46: 310 (1990).

Alternatively, monoclonal anti-CITED4-antibodies may be generated within the scope of the present invention. Monoclonal antibodies may be obtained by injecting mice with a composition comprising the inventive nucleic acid sequences inventive (regulating and/or encoding) nucleic acid sequences or the inventive amino acid sequences or the CITED4 polypeptide according to SEQ ID NO: 2, verifying the presence of antibody production by removing a serum sample, removing the spleen to obtain B lymphocytes, fusing the B-lymphocytes with myeloma cells to produce hybridomas, cloning the hybridomas, selecting positive clones which produce antibodies to the antigen (the inventive nucleic acid sequences or the inventive amino acid sequence or the CITED4 polypeptide according to SEQ ID NO: 2), culturing the clones that produce antibodies to the antigen, and isolating the antibodies from the hybridoma cultures. Alternatively, a monoclonal anti-CITED4-antibody according to the present invention may be derived from a human monoclonal antibody. Human monoclonal antibodies are typically obtained from transgenic mice that have been engineered to produce specific human antibodies in response to antigenic challenge. According to this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice may be used to produce human antibody-secreting hybridomas. Methods for obtaining human antibodies from transgenic mice are described, for example, by Green et al., Nature Genet. 7: 13 (1994), Lonberg et al., Nature 368: 856 (1994), and Taylor et al., Int. Immun. 6: 579 (1994). Monoclonal antibodies may be isolated and purified from hybridoma cultures by a variety of well-established techniques. Such isolation techniques include affinity chromatography with Protein-A Sepharose, size-exclusion chromatography, and ionexchange chromatography (see, for example, Coligan at pages 2.7.1-2.7.12 and pages 2.9.1-2.9.3; Baines et al., "Purification of Immunoglobulin G (lgG),"in Methods in Molecular Biology, Vo1.10, pages 79-104 (The Humana Press, Inc. 1992)).

Alternatively, an anti-CITED4-antibody may be derived from a "humanized" monoclonal antibody. Humanized monoclonal antibodies are usually produced by transferring mouse complementary determining regions from heavy and light variable chains of the mouse immunoglobulin into a human variable domain. Typical residues of human antibodies are then substituted in the framework regions of the murine counterparts. The use of antibody components derived from humanized monoclonal antibodies obviates potential problems associated with the immunogenicity of murine constant regions. General techniques for cloning murine immunoglobulin variable domains are described, for example, by Orlandi et al., Proc. Nat'l Acad. Sci. USA 86: 3833 (1989). Techniques for producing humanized monoclonal antibodies are described, for example, by Jones et al., Nature321: 522 (1986), Carter et al., Proc. Nat'l Acad. Sci. USA 89: 4285 (1992), Sandhu, Crit. Rev. Biotech. 12: 437 (1992), Singer et al., J Immun. 150: 2844 (1993), Sudhir (ed.), Antibody Engineering Protocols (Humana Press, Inc. 1995), Kelley, "Engineering Therapeutic Antibodies," in Protein Engineering: Principes and Practice, Cleland et al. (eds.), pages 399-434 (John Wiley & Sons, Inc. 1996), and by Queen et al., U.S. Patent No. 5,693,762 (1997).

Finally, rodent monoclonal antibodies to specific antigens may be obtained by methods known to those skilled in the art (see, for example, Kohler et al., Nature 256: 495 (1975), Coligan et al. (eds.), Current Protocols in Immunology, Vol. 1, pages 2.5.12.6.7 (John Wiley & Sons 1991) ["Coligan"], Picksley et al., "Production of monoclonal antibodies against proteins expressed in E. coli, "in DNA Cloning 2 : Expression Systems, 2nd Edition, Glover et al. (eds.), page 93 (Oxford University Press 1995)).

The present invention furthermore provides anti-idiotype antibodies, i.e. antibodies specifically recognizing inventive polyclonal or monoclonal antibodies as defined above. Polyclonal anti-idiotype antibodies may be prepared by immunizing animals with anti-CITED4-antibodies or antibody fragments, using standard techniques. See, for example, Green et al., "Production of Polyclonal Antisera," in Methods In MolecularBiology. Immunochemical Protocols, Manson (ed.), pages 1-12 (Humana Press 1992). Also, see Coligan at pages 2.4.1-2.4.7. Alternatively, monoclonal anti-idiotype antibodies may be prepared using anti-CITED4-antibodies or antibody fragments as immunogens with the techniques, described above. As another alternative, humanized anti-idiotype antibodies or subhuman primate anti-idiotype antibodies may be prepared using the above-described techniques. Methods for producing anti-idiotype antibodies are described, for example, by Irie, U.S. Patent No. 5,208,146, Greene, et.al., U.S. Patent No. 5,637,677, and Varthakavi and Minocha, J. Gen. Virol. 77: 1875 (1996).

For particular uses, it may be desirable to prepare fragments of anti-CITED4-antibodies, particular of any of the above anti-CITED4-antibodies. Such antibody fragments may be obtained, for example, by proteolytic hydrolysis of an inventive polyclonal, monoclonal (or anti-idiotypic) antibody as disclosed above. Proteolytic hydrolysis is usually obtained by pepsin or papain digestion of whole antibodies by conventional methods. As an illustration, antibody fragments may be produced by enzymatic cleavage of antibodies with pepsin to provide a fragment denoted F(ab')2. This fragment may be further cleaved using a thiol reducing agent to produce Fab' monovalent fragments. Optionally, the cleavage reaction may be performed using a blocking group for the sulfhydryl groups that result from cleavage of disulfide linkages. As an alternative, an enzymatic cleavage using pepsin produces two monovalent Fab fragments and an Fc fragment directly. These methods are described, for example, by Goldenberg, U.S. patent No. 4,331,647, Nisonoff et al., Arch Biochem. Biophys. 89 : 230 (1960), Porter, Biochem. J. 73: 119 (1959), Edelman et al., in Methods in Enzymology Vol.1, page 422 (Academic Press 1967), and by Coligan at pages 2.8.1-2.8.10 and 2.10.-2.10.4. Other methods of cleaving antibodies, such as separation of heavy chains to form monovalent light-heavy chain fragments, further cleavage of fragments, or other enzymatic, chemical or genetic techniques may also be used, so long as the fragments bind to the antigen that is recognized by the intact antibody. For example, Fv fragments comprise an association of VH and VL chains. This association may be non-covalent, as described by Inbar et al., Proc. Nat'1 Acad. Sci. USA 69: 2659 (1972). Alternatively, the variable chains may be linked by an intermolecular disulfide bond or cross-linked by chemicals such as glutaraldehyde (see, for example, Sandhu, Crit. Rev. Biotech. 12: 437 (1992)). These Fv fragments may comprise VH and VL chains which are connected by a peptide linker. These single-chain antigen binding proteins (scFv) are prepared by constructing a structural gene comprising DNA sequences encoding the VH and VL domains which are connected by an oligonucleotide. The structural gene is typically inserted into an expression vector which is subsequently introduced into a host cell, such as human or animal cells, e.g. brain cells. The recombinant host cells synthesize a single polypeptide chain with a linker peptide bridging the two V domains. Methods for producing scFvs are described, for example, by Whitlow et al., Methods A Companion to Methods in Enzymology 2: 97 (1991) (also see, Bird et al., Science 242: 423 (1988), Ladner et al., U.S. Patent No. 4,946,778, Pack et al., Biol Technology 11: 1271 (1993), and Sandhu, supra). As an illustration, a scFV may be obtained by exposing lymphocytes to a CITED4-polypeptide in vitro, and selecting antibody display libraries in phage or similar vectors (for instance, through use of immobilized or labeled CITED4-polypeptide). Genes encoding polypeptides having potential CITED4-polypeptide binding domains may be obtained by screening random peptide libraries displayed on phage (phage display) or on bacteria, such as E. coli. Nucleotide sequences encoding the polypeptides may be obtained in a number of ways, e.g. by random mutagenesis or random polynucleotide synthesis. These random peptide display libraries may be used to screen for peptides which interact with a known target which may be a protein or polypeptide, such as a ligand or receptor, a biological or synthetic macromolecule, or organic or inorganic substances. Techniques for creating and screening such random peptide display libraries are known in the art (Ladner et al., U.S. Patent No. 5,223,409, Ladner et al., U.S. Patent No. 4,946,778, Ladner et al., U.S. Patent No. 5,403,484, Ladner et al., U.S. Patent No. 5,571,698, and Kay et al., Phage Displayof Peptides and Proteins (Academic Press, Inc. 1996)) and random peptide display libraries and kits for screening such libraries are available commercially, for instance from CLONTECH Laboratories, Inc. (Palo Alto, CA), Invitrogen Inc. (San Diego, CA), New England Biolabs, Inc. (Beverly, MA), and Pharmacia LKB Biotechnology Inc. (Piscataway, NJ). Random peptide display libraries may be screened using the CITED4-sequences disclosed herein to identify proteins which bind to CITED4-polypeptide.

Another form of an antibody fragment is a peptide coding for a single complementarity-determining region (CDR). CDR peptides ("minimal recognition units") may be obtained by constructing genes encoding the CDR of an antibody of interest. Such genes are prepared, for example, by using the polymerase chain reaction to synthesize the variable region from RNA of antibody-producing cells (see, for example, Larrick et al., Methods: A Companion to Methods in Enzymology 2: 106 (1991), Courtenay-Luck, "Genetic Manipulation of Monoclonal Antibodies," in Monoclonal Antibodies : Production, Engineering and Clinical Application, Ritter et al. (eds.), page 166 (Cambridge University Press 1995), and Ward et al., "Genetic Manipulation and Expression of Antibodies", in Monoclonal Antibodies : Principles and Applications, Birch et al., (eds.), page 137 (Wiley-Liss, Inc. 1995)).

Pharmaceutical compositions are also encompassed herewith. Particularly, if inventive nucleic acid sequences, inventive (regulating and/or encoding) nucleic acid sequences and/or an inventive anti-CITED4 antibody, an inventive anti-idiotype antibody or antibody fragment thereof and/or inventive vectors as defined herein shall be applied as a medicament, these compounds are preferably formulated into a pharmaceutical composition. Preferably, inventive pharmaceutical compositions also comprise a pharmaceutically acceptable carrier, adjuvant or vehicle. In this context, a pharmaceutically acceptable carrier, adjuvant, or vehicle according to the invention refers to a non-toxic carrier, adjuvant or vehicle that does not destroy the pharmacological activity of the component(s) with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Pharmaceutical compositions of the present invention may be administered orally, parenterally, by inhalation spray, topically, rectally, nasally, buccally, vaginally or via an implanted reservoir. The term parenteral as used herein includes subcutaneous, intravenous, intramuscular, intra-articular, intra-synovial, intrasternal, intrathecal, intrahepatic, intralesional and intracranial injection or infusion techniques. Preferably, the pharmaceutical compositions are administered systemically, orally, intraperitoneally, intraarterially or intravenously. Sterile injectable forms of the pharmaceutical compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. The pharmaceutically acceptable compositions of this invention may be administered in any acceptable dosage form including, but not limited to, capsules, tablets, aqueous suspensions or solutions. The amount of the components of the present invention that may be combined with the carrier materials to produce a composition in a single dosage form will vary depending upon the host treated, the particular mode of administration. It has to be noted that a specific dosage and treatment regimen for any particular patient will depend upon a variety of factors, including the activity of the specific component employed, the age, body weight, general health, sex, diet, time of administration, rate of excretion, drug combination, and the judgment of the treating physician and the severity of the particular disease being treated. The amount of a component of the present invention in the composition will also depend upon the particular component(s) in the composition.

A further embodiment of the present application comprises a (diagnostic) method of detecting cancer of the central nervous system, preferably oligodendrogliomas, by detecting the expression of CITED4-nucleic acid sequences, preferably CITED4-RNA, in a biological sample, comprising the steps of:
(a) contacting inventive nucleic acid sequences as a probe molecule under stringent hybridizing conditions with either
   (i) a biological sample,
   (ii) RNA molecules isolated from the biological sample, or
   (iii) nucleic acid molecules synthesized from these RNA molecules, wherein these nucleic acid molecules hybridize under stringent conditions with an inventive nucleic acid sequence according to SEQ ID NO: 1, or its complementary strand;
(b) optionally amplifying the hybrids formed according to step a); and
(c) detecting the formation of hybrids of the inventive nucleic acid sequence and the RNA molecules or optionally their amplification products, wherein the presence of the hybrids or optionally their amplification products indicate expression of CITED4-nucleic acid sequences in the biological sample.

Probe molecules used according to the inventive method of detecting the expression of CITED4-nucleic acid sequences typically include inventive nucleic acid sequences as defined above, which may be double-stranded or single-stranded molecules, preferably single-stranded molecules. These probe molecules hybridize under stringent conditions to a nucleic acid sequence of CITED4 or an allelic variant thereof, wherein the nucleic acid sequence of CITED4 is preferably a (partial) sequence according to SEQ ID NO: 1 as defined above, an allelic variant thereof, or a complementary sequence thereof. Probe molecules may be DNA, RNA, oligonucleotides, and the like. Most preferably, inventive nucleic acid molecules for use in such an inventive method are selected from inventive nucleic acid sequences according to SEQ ID NO's: 3 to 14.

According to a first step of the inventive method, step a), inventive nucleic acid sequences are contacted as a probe molecule under stringent hybridizing conditions with RNA molecules isolated from a biological sample. Such a biological sample may be derived from human or animal origin and typically includes any e.g. body fluids including e.g. brain fluids, lymph fluids, blood, urine, etc., tissue samples (e.g. obtained by biopsy) including e.g. brain tissue, tumor tissue, e.g. cancers from the central nervous system including brain tumors, e.g. oligodendroglial tumors, samples derived from bones, connective tissue, bone marrow, as well as cell lysates derived from any human or animal tissue. If necessary, such biological samples may be prepared for subsequent analysis e.g. by preparing such cell lysates from biological samples or by solubilizing tissue or cells according to methods known in the art. Biological samples may contain RNA, or DNA molecules as well as proteins derived from a partial or a complete genomic sequence of CITED4 as defined above, optionally including promoter sequences of the genomic sequence of CITED4. RNA, or DNA molecules as well as proteins may be extracted from these biological samples by a skilled person by methods known in the art. By way of example, mRNA may be extracted from biological samples such as tissue samples, cell lysates etc., using methods such as the Proteinase K Method, the TRlzol^{®} method (Chomczynski P., Sacchi N., Anal Biochemistry 1987; 162:156-9), the Guanidine Isothiocyanate Method, the Silica Method (see e.g. Boom R. et al., J Clin Microbiol, 1990; 28:495-503), solid-phase techniques, etc..

A typical protocol for the Proteinase K method includes e.g. the following:. One hundred microliters containing a cell lysate or tissue sample (preferably minced) are treated with an equal volume of proteinase K (2 mg/ml in 20 mM Tris, pH 7.5, 10 mM EDTA, 0.1% SDS) at 45°C for 1 h. RNA may then be extracted with phenol-chloroform, precipitated with ethanol, and resuspended in 20 µl of DEPC-treated water prior to reverse transcription. After centrifugation, the pellet may be washed with 70% ethanol, air dried, and finally resuspended in 10 µl of DEPC treated water.

A typical protocol for the TRlzol^{®} method includes e.g. a commercially available mixture of guanidinium isothiocyanate acid and phenol chloroform (Life Technologies, Rockville, MD). For preparation of RNA one hundred microliters of serum or suspension of tissue are usually mixed with 800 µl of TRlzol^{®} LS reagent, 1 µl (10 mg) of yeast tRNA, and 200 µl of chloroform. The suspension of tissue is typically prepared by solubilizing tissue (biological sample) according to methods known in the art. The mixture is vortexed for 2 min, incubated for 20 min at room temperature, and centrifuged at 8,000 g for 20 min at 4°C. The upper phase needs to be precipitated with 600 µl isopropanol at-20°C for 18-24 hours. After centrifugation, the pellet may be washed with 70% ethanol, air dried, and finally resuspended in 10 µl DEPC treated water.

According to a typical guanidine isothiocyanate method, 100 µl of the biological sample are mixed with 1 µl of extraction solution (5.5 M guanidine isothiocyanate, 25 mM sodium citrate and sodium lauryl-sarcosine) and 1 µl (10 mg) of yeast tRNA, vortexed for 2 minutes and incubated for 30 minutes, at room temperature. This mixture is then centrifuged at 8,000 g at 4°C, for 5 minutes. The supernatant is typically transferred to another tube with 600 ml of isopropanol and incubated at -20°C for 18-24 hours. After centrifugation, the pellet may be washed with 70% ethanol, air dried, and finally resuspended in 10 µl DEPC treated water.

The silica method may be typically carried out according to the protocol described by Boom *et al.* (1990, supra). Therefore, one hundred microliters of each biological sample are usually added to a mixture of 30 µl of size-fractionated silica particles and 200 µl of lysis buffer L6 (8M guanidine isothiocyanate, 0.1M Tris-HCl (pH 6.4), 36 mM EDTA) and left at room temperature for 10 minutes, after which it was centrifuged at 8,000g for 30 seconds to sediment the nucleic acid-silica particle complexes. The pellet is typically washed once with 200 µl of washing buffer L2 (10 M guanidine isothiocyanate, 0.1 M Tris-HCl (pH 6.4)), twice with 500 µl of 70% (v/v) ethanol and once with 500 µl of acetone. The pellet is then dried at 56°C for 10 minutes and the nucleic acids eluted with 1µl of RNAsin (Life Technologies) in 60µl of water, at 56°C for 10 minutes. Subsequently, the tube is centrifuged at 8,000 g for 2 min to sediment the silica particles, and the supernatant, containing the RNA, may be used for further applications.

The contact of the inventive nucleic acid molecules with RNA molecules isolated from a biological sample typically occurs under stringent conditions. In this context, it is referred to the definition of "stringent conditions" as outlined above.

According to a further alternative of the inventive method for detecting the presence of CITED4-nucleic acid sequences in a sample from a patient, inventive nucleic acid sequences are contacted as a probe under stringent conditions with nucleic acid molecules synthesized from RNA molecules, which have been isolated from a biological sample as defined above. Preferably, these nucleic acid molecules synthesized from RNA molecules hybridize under stringent conditions with a (partial) nucleic acid sequence encoding CITED4 according to SEQ ID NO: 1, an allelic variant thereof, an inventive nucleic sequence, or their complementary strands. Synthesis of such a nucleic acid molecule may be carried out on basis of a RNA molecule isolated from a biological sample as defined above. Typically, such a nucleic acid molecule is a cDNA derived from the RNA molecule isolated from the biological sample. Methods for preparing cDNAs are well known in the art and may be reviewed in Sambrook *et al.* 2001, Molecular Cloning: A Laboratory Manual, Cold Spring Harbor, NY. Those methods include e.g. polymerase chain reaction (PCR), reverse transcriptase PCR (RT-PCR), primer extension reaction, etc.. Standard techniques for performing PCR are well-known (see, generally, Mathew (ed.), Protocols in Human Molecular Genetics (Humana Press, Inc. 1991), White (ed.), PCR Protocols - Current Methods and Applications (Humana Press, Inc. 1993), Cotter (ed.), Molecular Diagnosis of Cancer (Humana Press, Inc. 1996), Hanausek and Walaszek (eds.), Tumor Marker Protocols (Humana Press, Inc. 1998), Lo (ed.), Clinical Applications of PCR (Humana Press, Inc. 1998), and Meltzer (ed.), PCR in Bioanalysis (Humana Press, Inc. 1998)). As primers for cDNA synthesis any of the inventive nucleic acids may be used, preferably such nucleic acid sequences representing a segment of the CITED4 coding sequence. One variation of a PCR reaction for preparing cDNA molecules includes reverse transcriptase PCR (RT-PCR). In the RT-PCR technique, RNA typically is isolated from a biological sample as disclosed above, and reverse transcribed to cDNA. (see, for example, Wu et al. (eds.),"Rapid Isolation of Specific cDNAs or Genes by PCR", in Methods in Gene Biotechnology, pages 15-28 (CRC Press, Inc. 1997)).

According to an optional second step, step b), of the inventive method of detecting the presence of CITED4-nucleic acid sequences the hybrids formed according to step a) of the inventive method are eventually amplified in order to allow for a better detection of the formed hybrids and to increase sensitivity. Therefore, preferably, any of the above methods for amplification may be used, including PCR, RT-PCR, real-time PCR (RQ-PCR), primer extension methods, etc..

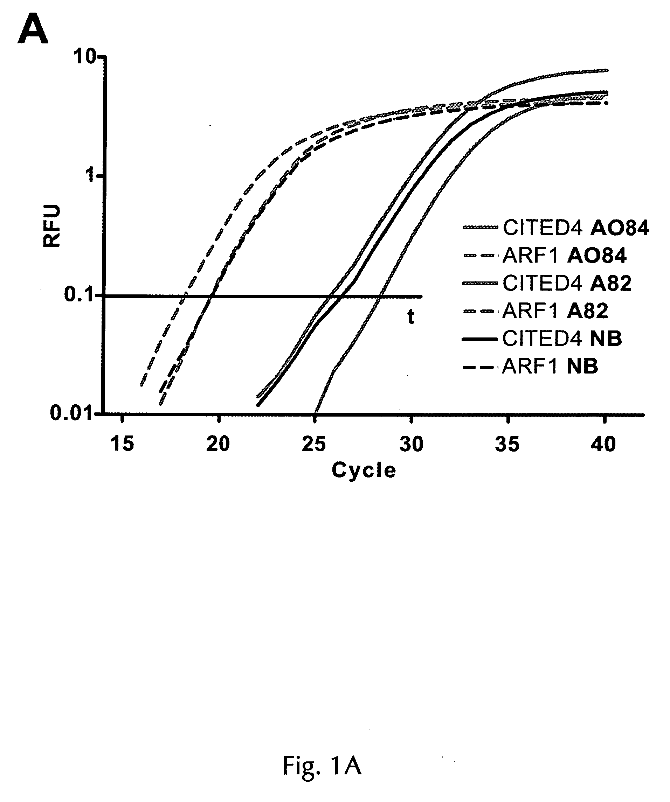

According to step c) of the inventive method as disclosed above, the formation of hybrids between the inventive nucleic acid sequences and either the RNA molecules isolated from a biological sample or the nucleic acid molecules synthesized on the basis of these RNA molecules, is detected, wherein the presence of the hybrids indicates the presence of CITED4 nucleic acids, e.g. RNA, in the biological sample. Detection of such hybrids is typically carried out by biophysical methods typically known by a skilled person. Therefore, the hybrids may stay intact in their hybrid structure (i.e. double stranded) or may be separated again from the inventive nucleic acid sequences (i.e. single stranded), e.g. by denaturing conditions. By way of example, PCR products may be fractionated by gel electrophoresis, and visualized by ethidium bromide staining. Alternatively, fractionated PCR products may be transferred to a membrane, hybridized with a detectably-labeled inventive nucleic acid sequence, and examined by autoradiography. Suitable labels for this purpose are indicated above. As an alternative approach, hybrids may be detected by prior labeling inventive nucleic acid sequences with fluorescence labels (see above), exciting the hybrids, formed in step a) of the inventive method, at the wavelength corresponding to their labels and detecting the signal via a fluorescence detection as widely known in the art. Most advantageously, such detection of hybrids may be carried out in combination with a real-time quantitative PCR (RQ-PCR), which additionally allows quantifying the amount of hybrids found in the biological sample. Therefore, preferably control samples are prepared, which reflect the background and allow setting a treshold value for optimal quantitative determination (see e.g. Valasek and Repa, Adv. Physiol. Educ. 29:151-159, 2005). Calculation of the determined CITED4 mRNA levels in each tumor can then be carried out in relation to a control sample after normalization of the obtained values. Such a procedure allows, particular with respect to methods such as PCR and RQ-PCR, a reliable quantification of the detected signals. A control sample in this context may be e.g. a non-neoplastic biological sample, such as a non-neoplastic brain tissue (BD Biosciences, St. Jose, CA). Furthermore, a normalization is typically carried out by using ADP-ribosylation factor 1 (ARF1, GenBank accession no. NM_001658) or actin-g (ACTG1, GenBank accession no. NM_001614) (Fig. 1A). Preferred sequences used for normalization include the following:

**Table 3: Inventive nucleic acid sequences according to SEQ ID NO: 35 to 38**

| **SEQ ID NO:** | **Primer** | **Sequence** | **Amplicon length** |
|---|---|---|---|
| 35 | ARF1-P1-for | 5'-gaccacgatcctctacaagc-3' | 111 bp |
| 36 | ARF1-P1-rev | 5'-tcccacacagtgaagctgatg-3' | |
| 37 | ACTG1-P1-for | 5'-cagctctcgcactctgttctt-3' | 163 bp |
| 38 | ACTG1-P1-rev | 5'-cgacgatggaaggaaacac-3' | |

Additional alternative approaches include the use of digoxigenin-labeled deoxyribonucleic acid triphosphates to provide chemiluminescence detection, and the C-TRAK colorimetric assay. Another approach for detection of CITED4 expression is the cycling probe technology, in which a single-stranded DNA target binds with an excess of DNA a RNA-DNA chimeric probe to form a complex. The RNA portion is then typically cleaved with RNAase H, and the presence of cleaved chimeric probe may be detected (see, for example, Beggs et al., J. Clin. Microbiol. 34: 2985 (1996), Bekkaoui et al., Biotechniques 20: 240 (1996)). Alternative methods for detection of hybrids or their single stranded nucleic acid sequences can utilize approaches such as nucleic acid sequence-based amplification, cooperative amplification of templates by cross-hybridization, and the ligase chain reaction (see, for example, Marshall et al., U.S. Patent No. 5,686,272 (1997), Dyer et al., J. Virol. Methods 60: 161 (1996), Ehricht et al., Eur. J. Biochem. 243: 358 (1997), and Chadwick et al., J. Tz Erol. Methods 70: 59 (1998)). In situ hybridization using inventive nucleic acids and cDNA/RNA isolated from biological samples may also be utilized to detect and to localize CITED4 gene expression in tissue samples. Methods for such in situ hybridization are well-known to those of skill in the art (see, for example, Choo (ed.), In Situ Hybridization Protocols (Humana Press, Inc. 1994), Wu et al. (eds.),"Analysis of Cellular DNA or Abundance of mRNA by Radioactive In Situ Hybridization (RISH)," in Methods in Gene Biotechnology, pages 259-278 (CRC Press, Inc. 1997), and Wu et al. (eds.),"Localization of DNA or Abundance of mRNA by Fluorescence In Situ Hybridization (RISH),"in Methods in Gene Biotechnology, pages 279-289 (CRC Press, Inc. 1997)). Various additional approaches are well-known to those of skill in the art (see, for example, Mathew (ed.), Protocols in Human Molecular Genetics (Humana Press, Inc. 1991), Coleman and Tsongalis, Molecular Diagnostics (Humana Press, Inc. 1996), and Elles, Molecular Diagnosis of Genetic Diseases (Humana Press, Inc., 1996)). Hybrids furthermore may be detected via their labels, i.e. any of the labels as defined above.

The present invention furthermore provides an alternative (diagnostic) method for detecting cancer of the central nervous system, preferably oligodendrogliomas, by detecting expression of CITED4-nucleic acid sequences, e.g. as defined by SEQ ID NO: 1, preferably a partial or a complete genomic sequence of CITED4-RNA or a CITED4-expression product, in a biological sample, comprising the steps of:
(a) contacting an inventive antibody or antibody fragment as defined above with either
   (i) a biological sample, or
   (ii) RNA molecules isolated from the biological sample, or
   (iii) nucleic acid molecules synthesized from these RNA molecules, or
   (iv) a CITED4-expression product; and
(b) detecting any of the bound antibody or bound antibody fragment.

In step a) of such an alternative method any antibody as mentioned above may be used, provided, that such antibody is capable of binding to either RNA molecules isolated from a biological sample, or to nucleic acid molecules synthesized from these RNA molecules. In this context, a biological sample is preferably as defined above. This alternative method allows to directly screen a biological sample as defined above, more preferably a cell lysate, or RNA molecules isolated from the biological sample, or nucleic acid molecules synthesized from these RNA molecules. A CITED4-expression product according to step a) may be a (poly)peptide derived from a partial or a complete genomic sequence of CITED4 as defined above, e.g. derived from an inventive nucleic acid sequence. Alternatively, a CITED4-expression product according to step a) may be an inventive amino acid sequence as defined above.

According to step b) of the above alternative method for detecting the presence of CITED4-nucleic acid sequences, the bound antibody or bound antibody fragment may be detected by a biophysical method as disclosed above. Most preferably, the antibody is detected by methods such as Western Blotting or Immunoblotting. Therefore, the sample containing the bound antibody or bound antibody fragment may be first resolved by gel electrophoresis. Methods for adequately preparing a gel electrophoresis are well known to a skilled person. E.g. the gel acrylamide concentration has to be chosen appropriately for the anticipated molecular weight of the bound antibody or bound antibody fragment to be detected. Transfer of the proteins fractionated by SDS-PAGE to a solid support membrane (Western blotting membrane) can be accomplished by either capillary blotting or by electroblotting (semi-dry and tank transfer systems). More preferably, the method of transfer is electroblotting. In this procedure, a sandwich of gel and solid support membrane (Nitrocellulose or PVDF) is compressed in a cassette and immersed in buffer between two parallel electrodes. A current is passed at right angles to the gel, which causes the separated proteins to migrate out of the gel and onto the solid support membrane. Once the bound inventive antibodies or antibody fragments have been transferred to the solid support membrane, the membrane is referred to as a "blot". Detection of the bound antibodies or the bound antibody fragments on the blot may occur directly subsequent to blotting by any of the biophysical methods as disclosed above. Therefore, the bound antibodies or the bound antibody fragments are preferably labelled. Alternatively, detection according to step b) may occur via utilization of a labelled (secondary) antibody. Therefore, the (primary) antibodies or antibody fragments may be bound subsequent to blotting by a labelled (secondary) antibody, preferably an anti-idiotype antibody as disclosed above, which is capable of specifically binding to the inventive (primary) antibodies or antibody fragments. The method of either detection is dependent upon the label that has been conjugated to the primary or secondary antibody. Generally, any of the labels mentioned above may be used for detection. The most common antibody label used in Western blotting is an enzyme such as alkaline phosphatase or horseradish peroxidase, which can be detected visually through the conversion of a colorimetric substrate (chromagen) to a colored precipitate at the site of antibody binding. Alternatively, chemiluminescent substrates may be employed which emit light upon conversion by the enzyme. The light emitted at the site of substrate conversion can be captured on x-ray film (via autoradiography). The appropriate working concentration of the primary antibody is typically dependent upon the binding characteristics of the primary antibody, but is also greatly affected by the type of detection system that is employed. If the proper primary antibody dilution for a colorimetric detection system is substituted into a chemiluminescence detection system without further optimization, it may occur that a background signal is observed. In such a case it is preferred to perform an additional dilution series with the primary antibody in order to determine the optimal dilution for this more sensitive detection system. Other labels suitable for the above mentioned alternative method for detecting the presence of CITED4-nucleic acid sequences include without being limited thereto; ¹²⁵I-labeled secondary antibody, which can be detected using a photographic film, ¹²⁵I-labeled Protein A (In this case Protein A is used instead of a secondary antibody, as it will bind to the Fc region of IgG molecules), Gold-labeled second antibody (The minute gold particles are directly visible as a red color when they are bound with the second antibody to the primary antibody) or a biotinylated secondary antibody (in this case the blot is incubated with the biotinylated secondary antibody, then incubated with enzyme-conjugated avidin that binds strongly to the biotin. This system will give an enhanced signal, as multiple biotin molecules can be attached to a single antibody molecule. The enzyme used is usually alkaline phosphatase or horseradish peroxidase).

The present invention also provides a (diagnostic) method for determining the degree of methylation of the complete or partial regulating and/or encoding sequence of CITED4 as defined above, e.g. as defined by SEQ ID NO: 39, in a biological sample, comprising the steps of:
(a) optionally isolating the complete or partial regulating and/or encoding sequence of CITED4 from a biological sample;
(b) treating the isolated regulating and/or encoding sequence of CITED4 with a deaminating agent;
(c) contacting the deaminated isolated regulating and/or encoding sequence of CITED4 with an inventive (regulating and/or encoding) nucleic acid sequence;
(d) amplifying the deaminated isolated regulating and/or encoding sequence of CITED4; and
(e) sequencing the amplified products from step d), and
(f) determining the methylation status by comparing the obtained sequence with the native, non-deaminated sequence of the regulating and/or encoding sequence of CITED4.

For purposes of the above (diagnostic) method the degree of methylation of the complete or partial regulating and/or encoding sequence of CITED4 is preferably determined within any segment (i.e. "complete or partial") of the regulating and/or encoding sequence of CITED4. More preferably, the complete or partial regulating and/or encoding sequence of CITED4 is (derived from) a sequence according to SEQ ID NO: 39, which also includes regulating sequences such as the promoter region of CITED4. With respect to segments of a complete or partial regulating and/or encoding sequence of CITED4 it is referred to the definitions as given above under inventive (regulating and/or encoding) nucleic acid sequences.

Therefore, according to a first step a), the complete or partial regulating and/or encoding sequence of CITED4 is optionally isolated from a biological sample, wherein the biological sample is preferably as defined above. Additionally, isolation of the biological sample may be carried out by a skilled person by any method known in the art (see e.g. Sambrook et al. 2001, Molecular Cloning: A Laboratory Manual, Cold Spring Harbor, NY). If present in a double stranded form, the (isolated) complete or partial regulating and/or encoding sequence of CITED4 may be separated into its single strands, e.g. by applying denaturing conditions (guanidiniumthiocyanate, urea, etc.) and/or by lowering the ionic strength of the buffer. Most preferably, the (isolated) complete or partial regulating and/or encoding sequence of CITED4 will be used in its single stranded form in order to avoid artifacts during subsequent deamination and analysis of the strands.

According to step b) the complete or partial regulating and/or encoding sequence of CITED4 is treated with a deaminating agent, preferably under weak acidic pH conditions (e.g. pH 4-6, preferably 5). As a deaminating agent e.g. sodium bisulfite (sodium hydrogen sulfite (NAHSO₃)) or 5-aza-2'-deoxycytidine may be used. By way of example, sodium bisulfite genomic sequencing may be carried out as described by Frommer *et al*. (Frommer, M. et al., Proc. Natl Acad. Sci. USA, 89, 1827-1831). Therefore, the DNA may be denatured e.g. in 111 µl of 0.3 M NaOH (in the presence of 10 µg of yeast mRNA) at 42°C for 20 min. Freshly prepared sodium bisulfite solution [1.2 ml, 541 g/l, or about 3 M (ACS grade reagent; Sigma, St Louis, MO)] e.g. in 10 mM hydroquinone pH 5.0, are added to the denatured DNA. The reaction may be overlaid with e.g. 200 µl of mineral oil (IR grade; Sigma) and incubated at 55°C for 4 h in the dark. Then, DNA will be desalted using e.g. the Wizard DNA purification kit (Promega, Madison, WI) and eluted in 105 µl of 1 mM Tris-HCl pH 8.5. One hundred microliters of this DNA solution are desulfonated in 0.3 M NaOH at 37°C for 30 min. For neutralization, ammonium acetate will be added to a final concentration of 3 M and the DNA is precipitated with ethanol using 1 µg of yeast mRNA as carrier. The DNA is dissolved in 100 µl of 10mM Tris-HCl pH 8.5 and stored at-20°C until further use.

In step c) the deaminated isolated complete or partial regulating and/or encoding sequence of CITED4 may be contacted with an inventive (regulating and/or encoding) nucleic acid sequence, preferably with (regulating and/or encoding) nucleic acid sequences according to SEQ ID NO's: 15 to 34, as disclosed in Table 2 above.

According to a further step d) the deaminated isolated complete or partial regulating and/or encoding sequence of CITED4 may be amplified, preferably according to standard protocols by using any of the amplification methods as mentioned above including PCR, RQ-PCR, primer extension, etc..

According to a step e), the amplified products as obtained according to step d) are sequenced. This step may be carried out by any method known in the art.

According to a final step f), the methylation status of the sequenced amplified products is determined. Therefore, the sequences of the PCR products are typically aligned to the corresponding genomic sequence, i.e. the (complete or partial) regulating and/or encoding sequence of CITED4 as defined above, more preferably the (complete or partial) regulating and/or encoding sequence of CITED4 derived from a sequence according to SEQ ID NO: 39. Such an alignment may be carried out by any method known in the art or as disclosed above in the section referring to "identity" of sequences. Subsequently, these sequences may be manually edited and further analyzed. Typically, cytosines in the genomic sequence that were converted to uracils and subsequently PCR-amplified as thymines correspond to unmethylated cytosines, whereas cytosines in the PCR products indicate the presence of 5-methylcytosines. This allows a direct determination of formerly methylated cytosines. The alignments may then be used (i) to calculate the average methylation level for each cytosine position; (ii) to visualize the methylation state of individual CpGs; and (iii) to determine the methylation density for each biological sample. To establish an association of the hypermethylation of the complete or partial regulating and/or encoding sequence of CITED4, e.g. according to SEQ ID NO: 39, with significantly longer progression free survival and overall survival of tumors of the CNS, Kaplan-Meier survival estimates may be established according to methods known in the art and as shown in the accompanying figures.

The above (diagnostic) method for determining the degree of methylation of the complete or partial regulating and/or encoding sequence of CITED4 may be applied with respect to any tumor as defined below, e.g. as selected from tumors of the central nervous system, such as brain tumors or oligodendroglial tumors, including gliomas, e.g. oligodendrogliomas, astrocytomas, ependymomas and/or mixed gliomas such as oligoastrocytomas, as well as neuroblastomas.

The biological function of CITED4 is to bind to the CREB - binding protein (CPB)/p300 as well as to the transcriptional coactivator EP300 (EA1-binding domain) via its CH1 domain. CITED4 thereby functions as a co-activator of transcription factor AP2. The transcriptional coactivator EP300 itself is involved in expression of hypoxia responsive genes and tumor angiogenesis. Angiogenes is typically referred to as the formation of new blood vessels, wherein tumor angiogenesis represents a particular form of angiogenesis and usually refers to growth of blood vessels from surrounding tissue to a solid tumor. If binding of CITED4 to the transcriptional coactivator EP300 is inhibited, the function of EP300 may be enhanced and its angiogenic promoting properties may be elevated. Any therapy, which is based on presence or absence of angiogenesis may thus either induce or inhibit activity of EP 300 by either promote or inhibit binding of CITED4 to the CREB - binding protein (CPB)/p300 as well as to the transcriptional coactivator EP300 (EA1-binding domain), e.g. by promoting or inhibiting translation of CITED4-mRNAs or expression of the CITED4-gene as defined above. Depending on the tumor type to be treated, it may be either required to suppress angiogenesis or to maintain (or even increase) angiogenesis. Typically, angiogenesis will be maintained or increased for gene therapy methods according to the present invention. Such a treatment may be carried out for any tumors as defined below. E.g. tumors, which are susceptible to radiotherapy or chemotherapy, may be eradicated by maintaining (or even increasing) the level of angiogenesis, e.g. by maintaining (or even increasing) EP300 activity. Such treatment may be particularly advantageous for treatment of brain tumors, gliomas e.g. oligodendroglial tumors. In these tumors, angiogenesis may lead to a different effect as in other tumor types, probably due to effects caused by the blood-brain barrier, which may lead to a different supply of the tumor with nutrients and substances necessary to promote cell growth. As an alternative approach, some tumors may require suppression of angiogenesis, e.g. by inhibiting EP300 activity. Then, angiogenesis may be lowered or even abolished by suppression of angiogenesis, e.g. by inhibiting EP300 activity.

Accordingly, the present invention provides as a further embodiment a method for gene-therapy suitable for treating or preventing cancers of the central nervous system, preferably oligodendrogliomas. Preferably, the inventive gene-therapy method comprises the step of introducing *in vivo* (or *in vitro*) either a (naked) inventive nucleic acid sequence, a (naked) inventive (regulating and/or encoding) nucleic acid sequence, a siRNA as defined above or an inventive vector comprising such nucleic acid sequences into cells, preferably cells as defined above. Therefore, the inventive vectors and/or the above nucleic acid sequences, are preferably capable to hybridize under cellular (stringent) conditions with the complete naturally occurring coding (or, if required, with the non-coding) sequence of CITED4, preferably according to SEQ ID NO: 1 or 39, or an allelic variant thereof, or a part of these sequences. Hybridization of said nucleic acid sequences may lead to an inactive (or, if required, to an active (=restored)) CITED4 gene, e.g. by incorporating these sequences into the sequence of CITED4. The incorporation of these sequences *in vivo* typically occurs via homolog recombination or insertional mutagenesis.

If provided in its naked form, an inventive nucleic acid sequence and/or an inventive (regulating and/or encoding) nucleic acid sequence may be used for gene-therapy. Preferably, such a sequence comprises a sequence, wherein preferably at least 1, 1-2, 1-3, 1-4, 1-5, 1-6, 1-7, 1-8, 1-9, 1-10, 1-15, or 1-20 nucleic acids have been deleted, substituted or inserted with respect to their native (= non-altered) sequences. Alternatively, a nucleic acid may be used containing the complete naturally occurring regulating and/or encoding (or, if required, the non-coding) sequence of CITED4, preferably according to SEQ ID NO: 1 or 39, an allelic variant thereof, wherein 1, 1-2, 1-3, 1-4, 1-5, 1-6, 1-7, 1-8, 1-9, 1-10, 1-15, 1-20, 1-50, 1-175, 1-100, 1-150, 1-200, 1-250, or 1-552 nucleic acids have been deleted, substituted or inserted with respect to their native (= non-altered) sequences. As a further alternative, a non-altered inventive nucleic acid sequence or an inventive (regulating and/or encoding) nucleic acid sequence may be used for gene-therapy purposes. Additionally, a non-altered complete (naturally occurring) regulating and/or encoding (or, if required, non-coding) sequence of CITED4, preferably according to SEQ ID NO: 1 or 39, an allelic variant thereof, or a part of these sequences, may be used for gene-therapy purposes. If provided in their naked form, the inventive nucleic acid sequences and/or an inventive (regulating and/or encoding) nucleic acid sequence may be part of a pharmaceutical composition.

According to another alternative, a siRNA may be provided as defined above for gene-therapy purposes. Therefore, the siRNA may be transferred into cells, preferably cells as defined above, either in its naked form or formulated with a pharmaceutically acceptable carrier (e.g. as a pharmaceutical composition). In vivo, siRNA typically causes degradation of the complementary cellular mRNA, and leads to the knockdown of gene activity. Accordingly, siRNA as used in the context of the present invention, is typically directed to a naturally occurring mRNA sequence derived from the CITED4 gene as defined above, e.g. according to SEQ ID NO: 1 or 39, or an allelic variant thereof, and/or a part of these sequences.

If a vector is provided for gene-therapy purposes, the vector typically comprises the same inventive nucleic acid sequences or inventive (regulating and/or encoding) nucleic acid sequences as provided in their naked from (see above). If the vector or (naked) nucleic acid sequence) as used for gene-therapy comprises a nucleic acid sequence, wherein the entire (coding or non-coding) gene of CITED4 or its allelic variant and/or its promoter region has been deleted, the inventive vector or the inventive nucleic acid sequences may additionally contain a contiguous stretch of the naturally occurring upstream and downstream sequences of the naturally occurring (coding or non-coding) gene of CITED4 or its allelic variant and/or its promoter region, in order to allow stringent hybridization of such nucleic acid sequence or vector under cellular conditions. Such naturally occurring upstream and downstream sequences may be readily determined by a skilled person by databank analysis on basis of the sequence of CITED4 (see above).

According to one preferred alternative, the herein defined inventive vectors or nucleic acids as utilized for gene-therapy may carry at least one of the following mutations, which correspond to naturally occurring polymorphisms of the CITED4 gene (see Table 4):

**Table 4: Polymorphisms occurring in the CITED4 gene**

| **No.** | **Polymorphism** |
|---|---|
| 1 | NC_000001:g.40997143G>A |
| 2 | NC_000001 :g.40997205C>G |
| 3 | NC_000001:g.40997259del1 |
| 4 | NC_000001 :g.40997260del 1 |
| 5 | NM_133467.2:c.-74C>G |
| 6 | NM_133467.2:c.27G>C (Glu9Asp) |
| 7 | NM_133467.2:c.54G>T (Pro18 Pro) |
| 8 | NM_133467.2:c.-72G>T |
| 9 | NM_133467.2:c.95T>A (Leu32Gln) |
| 10 | NM_133467.2:c.170G>C (Arg57Pro) |
| 11 | NM_133467.2:c.173A>G (Gln58Arg) |
| 12 | NM_133467.2:c.327-362del (PPGPQPAPSAAA110-121 del) |
| 13 | NM_133467.2:c.393C>T (Gly131Gly) |
| 14 | NM_133467.2:c.410T>G (Ile137Ser) |
| 15 | NM_133467.2:c.519G>T (Leu173Phe) |

For gene-therapy purposes, the inventive vectors, the above defined (naked) nucleic acids or a (mutagenized) CITED4 gene as defined above, may be transfected into cells by a variety of methods, e.g. by microinjection, lipid-mediated strategies or by viral-mediated strategies, by delivery of naked nucleic acid, use of a cationic lipid carrier with a nucleic acid molecule that encode the inventive vectors and nucleic acids or a (mutagenized)CITED4 gene as defined above. Additionally, the inventive vectors, inventive nucleic acids or a (mutagenized)CITED4 gene as defined above may be delivered using recombinant viral vectors, including for example, adenoviral vectors (e.g. Kass-Eisler et al., Proc. Nat'l Acad. Sci. USA 90: 11498 (1993), Kolls et al., Proc. Nat'l Acad Sci. USA 91: 215 (1994), Li et al., Hum. Gene Ther. 4: 403 (1993), Vincent et al., Nat. Genet. 5 : 130 (1993), and Zabner et al., Cell 75: 207 (1993)), adenovirus-associated viral vectors (Flotte et al., Proc. Nat'l Acad. Sci. USA 90: 10613 (1993)), alphaviruses such as Semliki Forest Virus and Sindbis Virus (Hertz and Huang, R Vir. 66: 857 (1992), Raju and Huang, J. Vir. 65: 2501 (1991), and Xiong et al., Science 243: 1188 (1989)), herpes viral vectors (e.g. U.S. Patent Nos. 4,769,331, 4,859,587, 5,288,641 and 5,328,688), parvovirus vectors (Koering et al., Hum. Gene Therap.5: 457 (1994)), pox virus vectors (Ozaki et al., Biochem. Biophys. Res. Comm. 193: 653 (1993), Panicali and Paoletti, Proc. Nat'l Acad. Sci. USA 79: 4927 (1982)), pox viruses, such as canary pox virus or vaccinia virus (Fisher-Hoch et al., Proc. Nat'l Acad. Sci. USA 86: 317 (1989), and Flexner et al., Ann. N. Y. Acad. Sci. 569: 86 (1989)), and retroviruses (e.g. Baba et al., J. Neurosurg 79: 729 (1993), Ram et al., Cancer Res. 53: 83 (1993), Takamiya et al., J. Neurosci. Res 33: 493 (1992), Vile and Hart, Cancer Res. 53: 962 (1993), Vile and Hart, Cancer Res. 53: 3860 (1993), and Anderson et al., U.S. Patent No. 5,399,346). Within various embodiments, either the viral vector itself, or a viral particle which contains the viral vector may be utilized herein.

As an illustration of one transfer system for gene-therapy, adenoviruses may be used. The adenovirus, a double-stranded DNA virus, represents a well-characterized gene transfer vector for delivery of a heterologous nucleic acid molecule (for a review, see Becker et al., Meth. Cell Biol. 43: 161 (1994); Douglas and Curiel, Science & Medicine 4: 44 (1997)). The adenovirus system offers several advantages including: (i) the ability to accommodate relatively large DNA inserts, (ii) the ability to be grown to high-titer, (iii) the ability to infect a broad range of mammalian cell types, and (iv) the ability to be used with many different promoters including ubiquitous, tissue specific, and regulatable promoters. In addition, adenoviruses may be administered by intravenous injection, because the viruses are stable in the bloodstream. Using adenovirus vectors where portions of the adenovirus genome are deleted, inserts are incorporated into the viral DNA by direct ligation or by homologous recombination with a co-transfected plasmid. In an exemplary system, the essential E1 gene is deleted from the viral vector, and the virus will not replicate unless the E1 gene is provided by the host cell. When intravenously administered to intact animals, adenovirus primarily targets the liver. Although an adenoviral delivery system with an E1 gene deletion cannot replicate in the host cells, the host's tissue will express and process an encoded heterologous protein. Host cells will also secrete the heterologous protein if the corresponding gene includes a secretory signal sequence. Secreted proteins will enter the circulation from tissue that expresses the heterologous gene (e.g. the highly vascularized liver). Moreover, adenoviral vectors containing various deletions of viral genes may be used to reduce or eliminate immune responses to the vector. Such adenoviruses are E1-deleted, and in addition, contain deletions of E2A or E4 (Lusky et al., J. Virol. 72: 2022 (1998); Raper et al., Human Gene-therapy 9: 671 (1998)). The deletion of E2b has also been reported to reduce immune responses (Amalfitano et al., J.Virol. 72: 926 (1998)). By deleting the entire adenovirus genome, very large inserts of heterologous DNA may be accommodated. Generation of so called "gutless" adenoviruses, where all viral genes are deleted, are particularly advantageous for insertion of large inserts of heterologous DNA (for a review, see Yeh. and Perricaudet, FASEBJ 11: 615 (1997)).

For gene-therapy purposes, high titer stocks of recombinant viruses capable of expressing an inventive vector, (naked) nucleic acid sequences as defined above or a mutagenized CITED4 gene as defined above, may be obtained from infected mammalian cells using standard methods. For example, recombinant HSV may be prepared in Vero cells, as described by Brandt et al., J. Gen. Virol. 72: 2043 (1991), Herold et al., J. Gen. Virol. 75: 1211 (1994), Visalli and Brandt, Virology 185: 419 (1991), Grau et al., Invest. Ophthalmol. Vis. Sci. 30: 2474 (1989), Brandt et al., J. Virol. Meth. 36: 209 (1992), and by Brown and MacLean (eds.), HSV Virus Protocols (Humana Press 1997).

Alternatively, the inventive vectors, (naked) nucleic acids as defined above or a mutagenized CITED4 gene as defined above may be introduced for gene-therapy purposes into a subject's cells by lipofection *in vivo* using liposomes. Synthetic cationic lipids may be used to prepare liposomes for *in vivo* transfection of a gene encoding a marker (Felgner et al., Proc. Nat'l Acad. Sci. USA 84: 7413 (1987); Mackey et al., Proc. Nat'l Acad. Sci. USA 85: 8027 (1988)). The use of lipofection to introduce exogenous genes into specific organs *in vivo* has certain practical advantages. Liposomes may be used to direct transfection to particular cell types, which is particularly advantageous in a tissue with cellular heterogeneity, such as the pancreas, liver, kidney, and, particularly preferred, brain. Lipids may be chemically coupled to other molecules for the purpose of targeting. Targeted peptides (e.g. hormones or neurotransmitters), proteins such as antibodies, or non-peptide molecules may be coupled to liposomes chemically.

Finally, electroporation represents another alternative mode of administration of the inventive vectors, (naked) nucleic acids as defined above or a mutagenized CITED4 gene as defined above for gene-therapy purposes. For example, Aihara and Miyazaki, Nature Biotechnology 16: 867 (1998), have demonstrated the use of *in vivo* electroporation for gene transfer into muscle. In an alternative approach to gene-therapy, a therapeutic gene may encode a CITED4 anti-sense RNA that inhibits the expression of CITED4.

The above disclosed inventive gene.therapy method be applied with respect to any tumor as defined below, e.g. as selected from tumors of the central nervous system, such as brain tumors or oligodendroglial tumors, including gliomas, e.g. oligodendrogliomas, astrocytomas, ependymomas and/or mixed gliomas such as oligoastrocytomas, as well as neuroblastomas.

The present invention also contemplates kits, preferably for performing an (diagnostic) assay for determining CITED4 gene expression. Such inventive kits typically comprise at least one container comprising inventive nucleic acids, inventive (regulating and/or encoding) nucleic acids, an anti-CITED4 antibody, an anti-idiotype antibody and/or antibody fragments as defined above. A kit may also comprise a second container comprising one or more indicator reagents capable of indicating the presence of inventive nucleic acids, inventive (regulating and/or encoding) nucleic acids, and/or a CITED4 antibody or antibody fragments as defined above. Examples of such indicator reagents include any detectable labels as defined above such as a radioactive label, a fluorescent label, a chemiluminescent label, an enzyme label, a bioluminescent label, colloidal gold, and the like. An inventive kit may also comprise a means for conveying to the user that inventive nucleic acids and/or inventive antibodies or antibody fragments are used to detect CITED4 protein. For example, written instructions may state as to how the enclosed inventive nucleic acids and/or antibody or antibody fragment may be used to detect CITED4. The written material may be applied directly to a container, or the written material may be provided in the form of a packaging insert.

According to another embodiment, the present invention includes the use of any of the above disclosed inventive nucleic acid sequences, inventive (regulating and/or encoding) nucleic acids, amino acid sequences, antibodies vectors, as well as kits for the preparation of a pharmaceutical composition for treating tumors of the central nervous system, such as brain tumors or oligodendroglial tumors, including gliomas, e.g. oligodendrogliomas, astrocytomas, ependymomas and/or mixed gliomas such as oligoastrocytomas, as well as neuroblastomas.

Also contemplated is the use of any of the above disclosed inventive nucleic acid sequences, inventive (regulating and/or encoding) nucleic acids, amino acid sequences, antibodies vectors, as well as kits for gene-therapy (purposes), preferably for gene-therapy (purposes) as outlined above.

Having now fully described this invention, it will be appreciated by those skilled in the art that the same can be performed within a wide range of equivalent parameters, concentrations and conditions without departing from the spirit and scope of the invention and without undue experimentation. While this invention has been described in connection with specific embodiments thereof, it will be understood that it is capable of further modifications. This application is intended to cover any variations, uses or adaptations of the invention following, in general, the principles of the invention and including such departures from the present disclosure as come within known or customary practice within the art to which the invention pertains and as may be applied to the essential features herein before set forth as follows in the scope of the appended claims.

All references cited herein, including journal articles or abstracts, published or unpublished patent applications, issued patents or any other references, are entirely incorporated by reference herein, including all data, tables, figures and text presented in the cited references. Additionally, the entire contents of the references cited within the references cited herein are also entirely incorporated by reference. Reference to known method steps, conventional methods steps, known methods or conventional methods is not any way an admission that any aspect, description or embodiment of the present invention is disclosed, taught or suggested in the relevant art.

### FIGURES

- **Fig. 1A:**: Reduced expression of CITED4 mRNA expression in a glioma with LOH 1p/19q (AO84) as compared to a glioma without LOH 1 p/19q (A82) and non-neoplastic brain tissue (NB). Shown are results obtained by real-time reverse transcription-PCR. Abscissa, cycle number; Ordinate, relative amount of PCR product. Note that the curves for A82 and NB are nearly identical for both the reference transcript ADP-ribosylation factor 1 (ARF1) and CITED4, In AO84, the ARF1 curve is shifted to the left side and the CITED4 curve is shifted to the right side relative to the respective curves for A82 and NB, indicating reduced expression of CITED4 in AO84. The calculated CITED4 mRNA level in AO84 was 20% of the level in NB and A82, respectively.

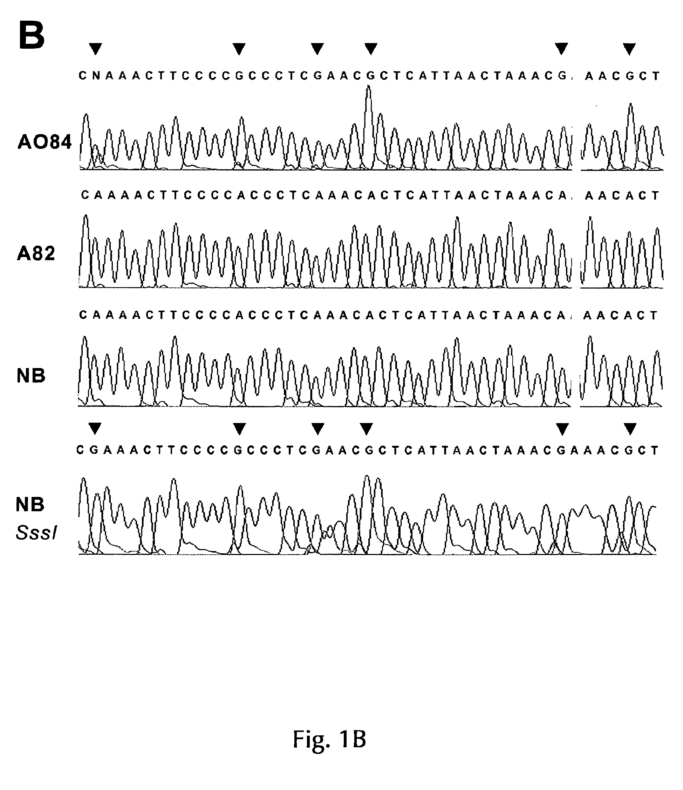

- **Fig. 1B**: Sequencing of parts of the CITED4 promoter region after sodium bisulfite treatment of DNA revealed methylation of CpG sites in AO84 (arrowheads in upper lane) but not in A82 and NB (middle lanes). The lower lane shows a positive control of DNA methylated in vitro using SssI methylase (shown is the reverse sequence from nucleotides g.40997168 to g.40997212, GenBank accession no. NC_000001) (see also SEQ ID NO: 39).

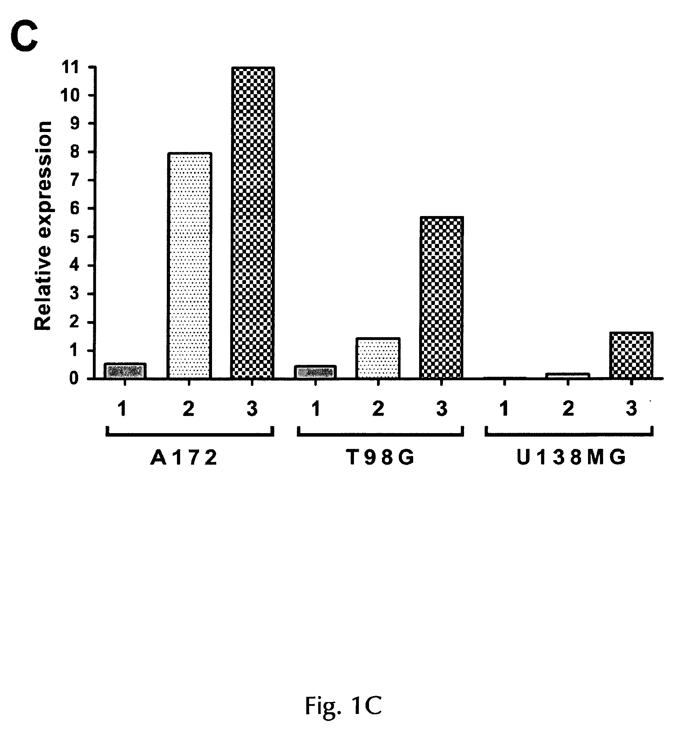

- **Fig. 1C:**: Induction of CITED4 mRNA expression in A172, T98G and U138MG glioma cells by 5- aza-2'-deoxycytidine and trichostatin A treatment (1, untreated cells; 2, 500 nM 5-aza-2'- deoxycytidine for 48h plus 1 µM trichostatin for 24 h; 3, 1 µM 5-aza-2'-deoxycytidine for 72h plus 1 µM trichostatin for 24 h).

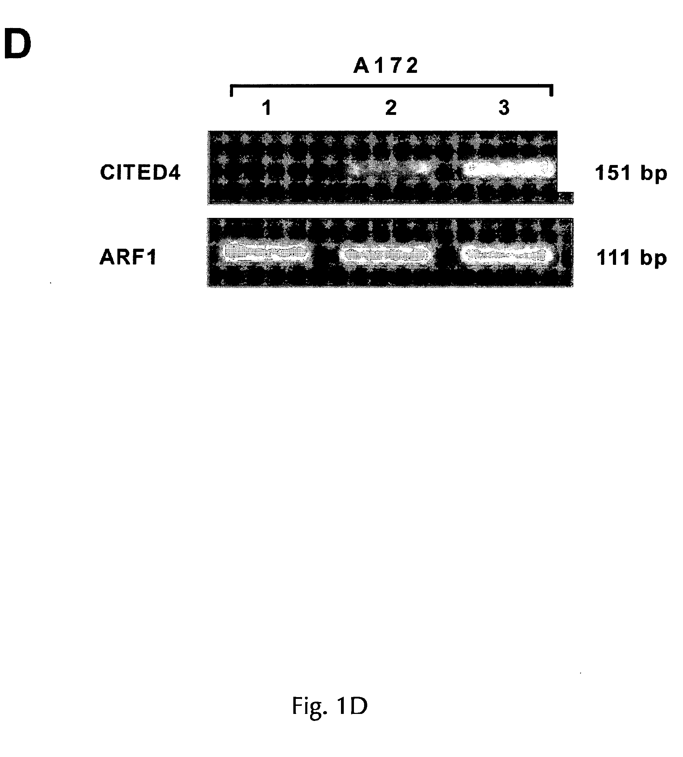

- **Fig. 1D:**: Results of reverse transcription-PCR analysis of CITED4 expression in A172 cells before (1) and after treatment with 5-aza-2'-deoxycytidine and trichostatin A under two different conditions (see above). Note marked induction of mRNA levels after treatment (bp = size of the respective PCR product in base pairs).

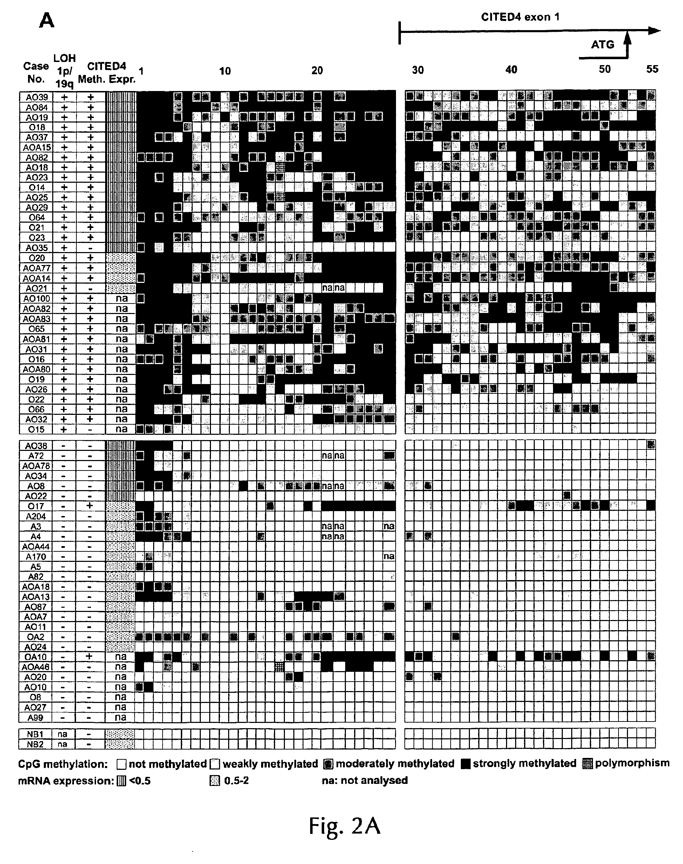

- **Fig. 2A:**: Schematic representation of methylation patterns in the 5'-CpG region of CITED4 in 62 gliomas, including 35 tumors with LOH 1p/19q and 27 tumors without LOH 1p/19q. In addition, findings in 2 samples of non-neoplastic brain tissue (NB1, NB2) are shown. Methylation at each of the 54 investigated CpG sites in the CITED4 promoter region was determined by sequencing of sodium bisulfite-treated DNA. The results are represented in a 4-tiered semiquantitative grey-scale pattern as indicated below the figure. CITED4 mRNA expression (Expr.) was determined by real-time reverse transcription-PCR analysis. Expression levels relative to non-neoplastic brain tissue (NB) were subdivided into 2 groups as shown below the figure. The location of exon 1 and the start codon (ATG) are indicated on top of the figure. Note a close association between LOH 1 p/19q and CITED4 hypermethylation in oligodendroglial tumors. Futhermore, 16/19 tumors with hypermethylation showed CITED4 mRNA levels of ≤ 0.5-fold relative to NB, as compared to 7/22 tumors without hypermethylation.
- **Figg. 2B-E:**: Significant correlations (log rank tests) of CITED4 promoter hypermethylation with progression-free survival (B, D) and overall survival (C, E) in patients with oligodendroglial tumors. Shown are Kaplan-Meier survival curves obtained for 45 patients (B,C), including 19 patients with WHO grade II and 26 patients with WHO grade III tumors, as well as in the subgroup of 26 patients with WHO grade III tumors (D,E).

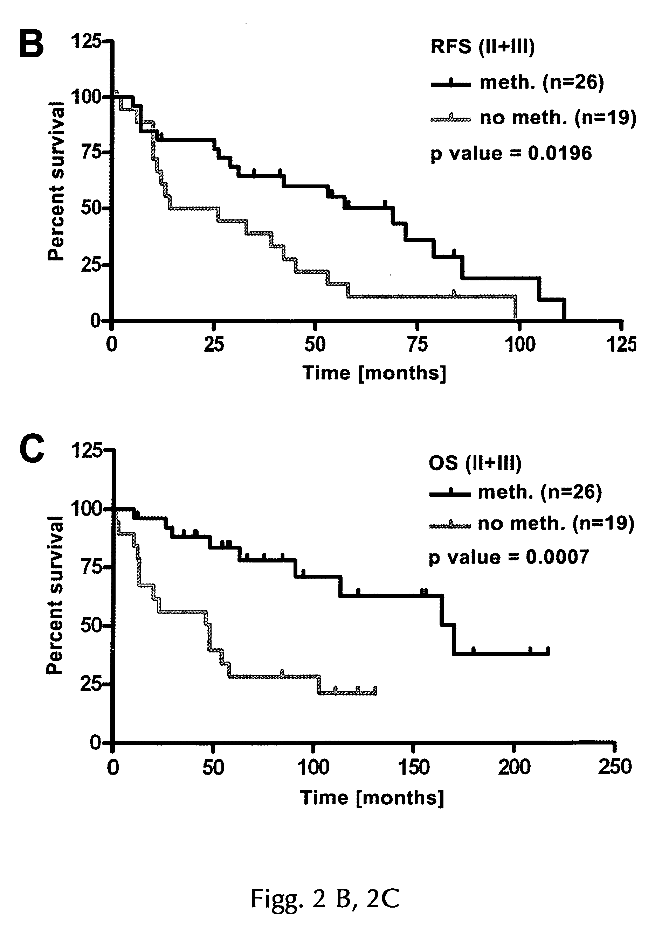

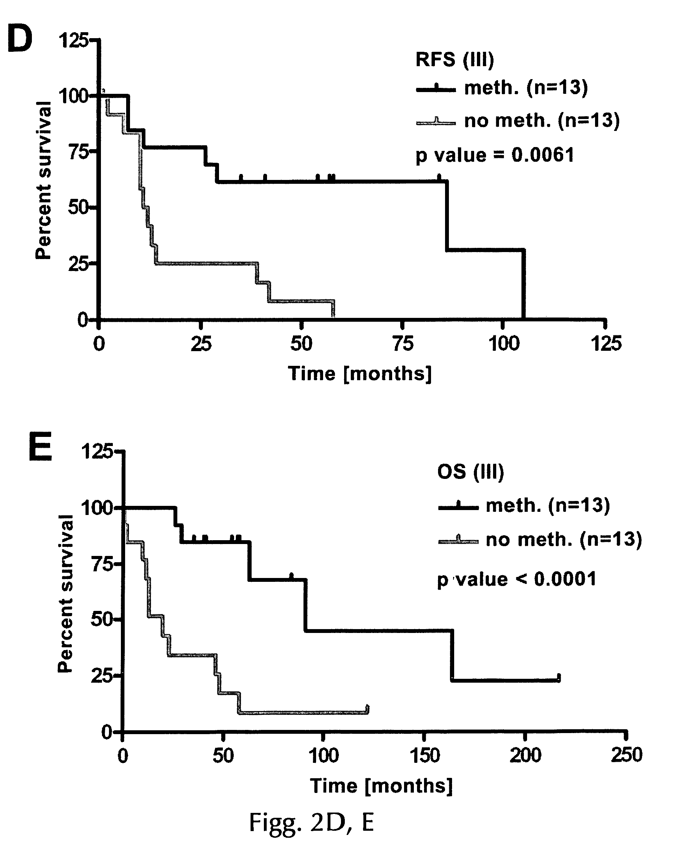

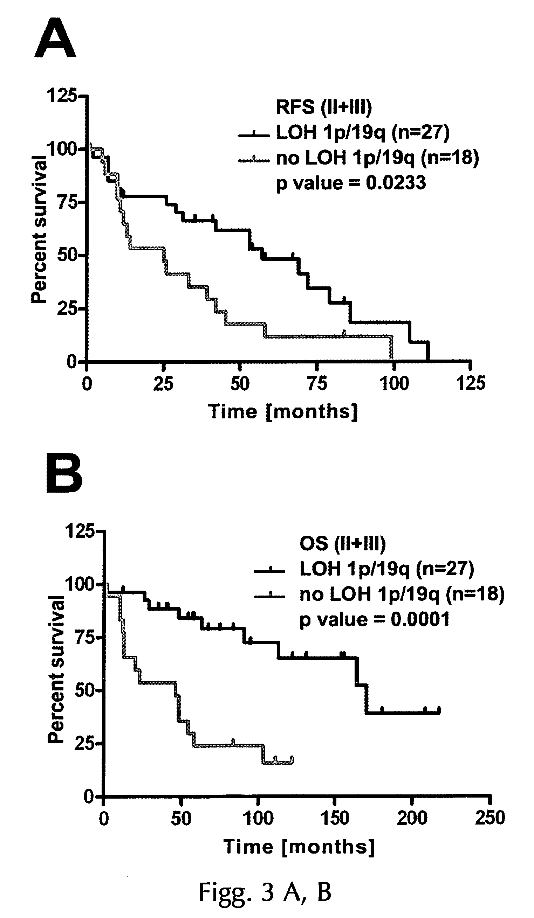

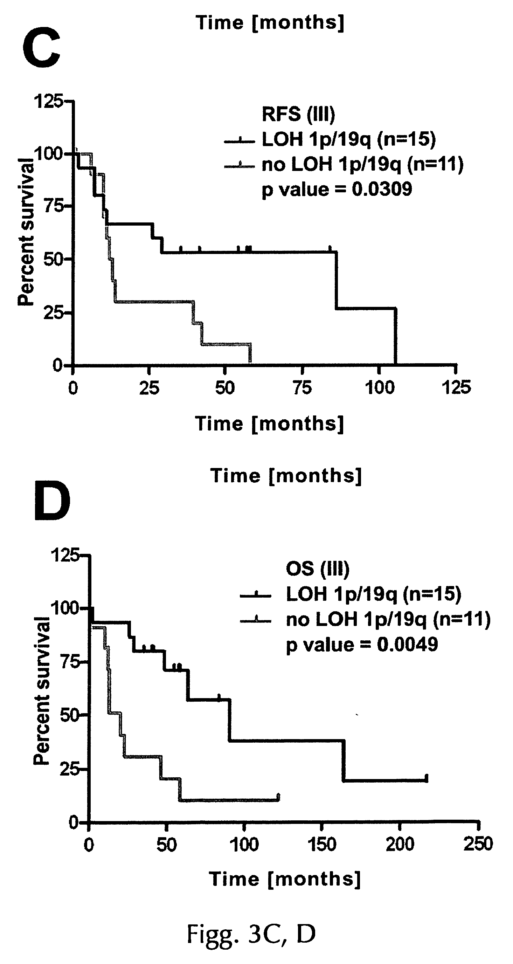

- **Fig. 3A-D:**: Kaplan-Meier survival curves for LOH 1 p/19q in the same patient series as investigated for CITED4 promoter methylation (see Fig. 2B-E). Note significant correlations (log rank tests) of LOH 1 p/19q with recurrence-free survival (RFS) and overall survival (OS). Shown are Kaplan-Meier survival curves obtained for 45 patients (A, B), including 19 patients with WHO grade II and 26 patients with WHO grade III tumors, as well as for the subgroup of 26 patients with WHO grade III tumors (C, D).

### Examples

### 1. cDNA microarray-based expression profiling

To identify novel candidate genes that are differentially expressed between gliomas with and without LOH 1p/19q, we performed cDNA microarray-based expression profiling on 42 gliomas, including 10 World Health Organization (WHO) grade II oligodendrogliomas with LOH 1p/19q, 10 WHO grade II diffuse astrocytomas without LOH 1p/19q, as well as 22 WHO grade III anaplastic oligodendrogliomas and oligoastrocytomas, half with and half without LOH 1 p/19q. DNA and RNA were extracted from frozen tumor tissue as reported elsewhere (van den Boom, J. et al., Am J Pathol 2003;163:1033-43). At least 10 microsatellite loci on 1 p and 6 microsatellite loci on 19q were analyzed for LOH in each tumor (Felsberg J. et al., Brain Pathol 2004; 14:121-30). For gene expression profiling, we used cDNA microarrays containing approximately 7,000 distinct human gene-specific fragments (each spotted in triplicate), with particular enrichment for cancer-relevant genes and genes located on 1 p and 19q. RNA amplification as well as microarray hybridization, stringent washing, scanning, and bioinformatic data analysis were performed according to standard techniques (see e.g. Wrobel, G. et al., Int J Cancer 2005;114:249-56). To identify genes whose expression discriminates between gliomas with and without LOH 1p/19q, we performed a bootstrapping algorithm based on a permutative student's t-test called Significance Analysis of Microarrays (SAM) (Tusher, V.G. et al., Proc Natl Acad Sci USA 2001; 98:5116-21). This algorithm was used to preselect genes showing significant differential expression, which were subsequently analyzed with the Nearest Shrunken Centroid method (PAM) as described by Tibshirani *et al.* (Tibshirani, R. et al., Proc Natl Acad Sci USA 2002;99:6567-72). Microarray-based expression profiling revealed a set of genes on 1 p that showed significantly lower expression in the gliomas with combined LOH 1p/19q (for details see Tews *et al.* 2005, submitted). Among these was the CREB-binding protein (CBP)/p300-interacting transctivator with glutamic acid [E]/aspartic acid [D]-rich carboxyl-terminal domain 4 gene (CITED4) at 1 p34.2 (OMIM 606815). CITED4 encodes a 184-amino acid protein of 24.5 kDa that binds to CPB as well as the putative tumor suppressor protein EP300 (E1A-binding protein, 300 kDa), and functions as a co-activator of the transcription factor AP2 (Braganca, J. et al., J Biol Chem 2002;277:8559-65). More recently, CITED4 was shown to block binding of hypoxia-inducible factor 1 alpha (HIF1a) to EP300 in vitro and to inhibit HIF1 a transactivation as well as hypoxia-mediated reporter gene activation in breast cancer cells (Fox, S.B. et al., Cancer Res 2004;64:6075-81). The same study implicated nuclear loss or cytoplasmic translocation of CITED4 in breast cancer development.

### 2. Differential mRNA expression by using real-time reverse transcription-PCR analysis

To evaluate the role of CITED4 in human gliomas, we first verified its differential mRNA expression by using real-time reverse transcription-PCR analysis on a series of 41 gliomas (exemplary PCR primer sequences used herein are provided in Tables 1 and 2 above). Twenty-one of these tumors showed LOH at all informative loci on 1p and 19q indicative of loss of one allele at the CITED4 locus (1p34.2). The other 20 gliomas either completely lacked LOH 1p/19q or showed LOH 1p restricted to loci on 1p36, i.e., distal to CITED4 (n=4). The CITED4 mRNA level in each tumor was calculated in relation to non-neoplastic brain tissue (BD Biosciences, St. Jose, CA) after normalization to ADP-ribosylation factor 1 (ARF1, GenBank accession no. NM_001658) or actin-g (ACTG1, GenBank accession no. NM_001614) (Fig. 1 A). The real-time reverse transcription-PCR experiments demonstrated CITED4 mRNA levels reduced by at least 50% relative to non-neoplastic brain tissue in 17 of the 21 gliomas (81 %) with loss of one CITED4 allele as opposed to 5 of 20 gliomas (25%) without allelic loss at the CITED4 locus (Fig. 2). Statistical analyses revealed that CITED4 mRNA levels were significantly lower (student's t-test, p < 0.001) and more commonly reduced (Fisher's exact test, p < 0.001) in gliomas with loss of one CITED4 allele.

### 3. Mutation analysis of CITED4

We then performed a mutation analysis of CITED4 in 47 gliomas, including 23 tumors with LOH 1 p/19q. For this purpose, we amplified the entire coding sequence of CITED4 in 5 overlapping fragments using PCR with genomic DNA as template. Four of these fragments were screened by single strand conformation polymorphism (SSCP) analysis while the fifth was directly sequenced. We detected a total of 15 different CITED4 sequence polymorphisms, i.e., sequence variations that were present in both tumor and blood DNA of the respective patients (Table 3, above). Most of these polymorphisms were single nucleotide exchanges. A 36-base pair in-frame deletion polymorphism (c.327-362del, GenBank accession no. NM_133467.2) was found in two patients, with one patient being constitutionally homozygous for this particular polymorphism. However, we did not detect any tumor-associated somatic mutations, thus indicating that structural alterations of this gene either are absent or very rare in gliomas.

### 4. Aberrant methylation of the CITED4 promoter region

Since the genomic sequence of CITED4 is markedly enriched in CpG sites, we next investigated the role of aberrant methylation of the CITED4 promoter region in gliomas. For this purpose, a total of 62 gliomas, including the 41 tumors studied for CITED4 mRNA expression, were subjected to DNA methylation analysis by sequencing the genomic region from nucleotides 40,996,844 to 40,997,411 within the CITED4 promoter region (Genbank accession no. NC_000001) (and SEQ ID NO: 39) after sodium bisulfite modification of the DNA (Herman J.G. et al., Proc Natl Acad Sci USA 1996, 93:9821-6). Hypermethylation, i.e., methylation of more than 50% of the 54 CpG sites investigated in the CITED4 promoter, was observed in 31 out of 35 gliomas with LOH 1p/19q (89%) but only 2 out of 27 gliomas without LOH 1p/19q (7%) (Figs. 1B and 2A) (Fisher's exact test, p < 0.0001). DNA extracted from non-neoplastic brain samples obtained at autopsy from two different individuals also lacked CITED4 hypermethylation (Fig. 2A). CITED4 mRNA levels were reduced by at least 50% relative to non-neoplastic brain tissue in 16 of 19 tumors (84%) with CITED4 promoter hypermethylation and 7 of 22 tumors (32%) without CITED4 promoter hypermethylation. Statistical analyses showed that CITED4 mRNA levels were significantly lower (student's t-test, p < 0.01) and more commonly reduced (Fisher's exact test, p < 0.01) in gliomas with CITED4 promoter hypermethylation. To further substantiate this association, three glioma cell lines (A172, T98G, U138MG) with CITED4 hypermethylation and low expression were treated with the demethylating agent 5-aza-2'-deoxycytidine in combination with the histone deacetylase inhibitor trichostatin A (Fig. 1C-D). All three cell lines demonstrated a marked increase in CITED4 mRNA levels after treatment (Fig. 1C-D). Sequencing of sodium bisulfite modified DNA from the A172 cell line showed at least partial demethylation of the CITED4 promoter sequence after demethylating treatment (data not shown).

### 5. Correlation between CITED4 promoter hypermethylation and patient survival

To assess the correlation between CITED4 promoter hypermethylation and patient survival, we performed univariate and multivariate statistical analyses for a series of 45 patients with oligodendroglial tumors (19 WHO grade II, 26 WHO grade III) and available clinical follow-up data (Felsberg *et al.,* 2004, supra). The median follow-up time of the patients after diagnosis was 122 months. Twenty-three patients died of their tumors while 22 patients were still alive at last follow-up. Median overall survival time after diagnosis was 103 months, median recurrence-free survival was 42 months. All patients were treated by neurosurgical tumor resection. Twenty-four of the 26 patients with WHO grade III tumors additionally received adjuvant therapy, comprising radiotherapy in 16 patients, alkylating chemotherapy in 1 patient, and combined radio- and chemotherapy in 7 patients. Kaplan-Meier survival estimates revealed that CITED4 promoter hypermethylation was associated with significantly longer progression free survival and overall survival in the entire group of 45 patients as well as in the subgroup of 26 patients with WHO grade III tumors (Fig. 2B-E). Similarly, significant correlations were found between LOH 1p/19q and survival data (Fig. 3). Multivariate analysis using step-wise Cox regression identified CITED4 hypermethylation and combined LOH 1 p/19q as related prognostic factors independent from WHO grade and patient age (see Tables 5A and 5B).

**Tables 5A and 5B: Results of multivariate Cox proportional hazards regression analysis. (*Hazard ratios and confidence intervals (95% Cls) are computed for a 10-year increment in age, for comparing WHO grade II versus WHO grade II, for comparing CITED4 promoter methylation status positive versus negative, or for comparing LOH 1p/19q versus no LOH 1p/19q. P values are computed for the Wald test statistics. RFS, recurrence-free survival; OS, overall survival.)**

| **Table 5A** | | | | |
|---|---|---|---|---|
| Factor | RFS Hazard ratio (95% Cl)* | p value | OS Hazard ratio (95% Cl)* | p value |
| Age at operation | 1.39 (1.07 - 1.80) | 0.01 | 1.26 (0.93 - 1.71) | 0.13 |
| WHO grade | 1.78 (0.87 - 3.61) | 0.11 | 3.09 (1.23 - 7.76) | 0.02 |
| CITED4 promoter methylation status | 0.34 (0.17 - 0.70) | 0.003 | 0.25 (0.10 - 0.62) | 0.003 |

| **Table 5B** | | | | |
|---|---|---|---|---|
| Factor | RFS Hazard ratio (95% Cl)* | p value | OS Hazard ratio (95% Cl)* | p value |
| Age at operation | 1.36 (1.05 - 1.76) | 0.02 | 1.24 (0.92 - 1.67) | 0.16 |
| WHO grade | 1.79 (0.88 - 3.62) | 0.11 | 3.33 (1.33 - 8.29) | 0.01 |
| LOH 1p/19q status | 0.37 (0.18 - 0.75) | 0.006 | 0.20 (0.08 - 0.51) | < 0.001 |

The statistical analyses as displayed in Tables 5A and 5B were performed using the statistical software package R, version 2.1.1 (R Development Core Team, 2005: A language and environment for statistical computing. R Foundation for Statistical Computing, Vienna, Austria. ISBN 3-900051-07-0, http://www.R-project.org). Because CITED4 promoter hypermethylation and LOH 1 p/19q were highly correlated, two separate models were calculated, either including the CITED4 promoter methylation status (Table 5A) or the LOH 1p/19q status (Table 5B), respectively, each together with WHO grade and patient age at operation.

### 6. Conclusion and advantages of the present invention

Combined LOH on 1 p and 19q appears to represent a hallmark for oligodendroglioma (Smith *et al.,* 2000, supra), which has emerged as prognostic marker of longer survival and better chemotherapeutic response in patients with anaplastic oligodendroglioma (Cairncross *et al.,* 1998, supra; Ino *et al.,* 2001, supra; Smith *et al.,* 2000, supra). Due to frequent LOH in oligodendroglioma and according to the classical concept of tumor suppressors, both regions are supposed to harbor potential tumor suppressor genes whose down-regulation might be responsive for tumor development and progression (Kleihues, P. et al., J Neuropathol Exp Neurol 2002; 61:215-25; discussion 26-9). Some studies suppose, that there is more than a single tumor suppressor gene on 1 p which may be involved in the pathogenesis of oligodendroglioma (Husemann *et al.,* 1999, supra). Identification of genes involved in tumorigenesis would improve decoding the underlying molecular mechanisms. Our study compares different glioma entities regarding gene transcription of the candidate regions harboring putative tumor suppressors. We recently narrowed down and fine mapped the critical regions on chromosomes 1 and 19 (Felsberg *et al.,* 2004, supra). We now screened these regions for tumor-relevant genes by using custom-made microarrays comprising gene-specific cDNAs fine mapping 1p36.31-13 and 19q13.2-33 besides a large set of genome-wide gene specific fragments with particular enrichment for cancer relevant genes.

The inventive integrated strategy combining microarray-based expression profiling with focused molecular analysis of a selected candidate gene now revealed frequent promoter hypermethylation and transcriptional down-regulation of CITED4 in oligodendroglial tumors with LOH 1p/19q. The close association between LOH 1p/19q and CITED4 promoter hypermethylation indicates that bi-allelic inactivation of CITED4 in these tumors is commonly achieved by loss of one allele and epigenetic silencing of the other allele. Loss of CITED4 expression could possibly reduce glioma cell growth by promoting the activity of the putative tumor suppressor protein EP300 (Iyer N.G. et al., Oncogene 2004;23:4225-31) and by elevating angiogenesis in these tumors. EP300 has been implicated in the regulation of several tumorigenic pathways, such as the transforming growth factor beta (TGF-b), retinoblastoma protein (pRb) and TP53 pathways (Iyer N.G. et al., Oncogene 2004; 23:4225-31). In particular, EP300 is crucially involved in the regulation of both TP53 protein stability and transcriptional activity (Grossman S.R., Eur J Biochem 2001; 268:2773-8). Since oligodendroglial tumors with LOH1p/19q usually lack TP53 mutations (Reifenberger G. et al., J Neuropathol Exp Neurol 2003; 62:111-26), inactivation of CITED4 may represent an alternative molecular mechanism by which these tumors escape from TP53-dependent control of proliferation and apoptosis. Summarizing the above, the present invention reveals advantageously detection of CITED4-nucleic acids, e.g. as derived from SEQ ID NO: 39, and detection of its expression and hypermethylation as a clinically useful prognostic marker in oligodendroglial tumors which is closely linked to LOH 1 p/19q and associated with longer patient survival.

## Claims

1. Nucleic acid sequence, selected from one of the following sequences:
a) a nucleic acid sequence according to SEQ ID NO: 1, or an allelic variation thereof, and having 10 to 200, 10 to 150, 10 to 100, 10 to 50, 10 to 40 or 10 to 30 nucleic acids in length;
b) a nucleic acid sequence complementary to a nucleic acid sequence according to SEQ ID NO: 1, or an allelic variation thereof, and having 10 to 200, 10 to150, 10 to 100, 10 to 50, 10 to 40 or 10 to 30 nucleic acids in length;
c) a nucleic acid sequence hybridizing under stringent conditions to a nucleic acid sequence according to SEQ ID NO: 1, or an allelic variation thereof, and having 10 to 200, 10 to 150, 10 to 100, 10 to 50, 10 to 40 or 10 to 30 nucleic acids in length;
d) a nucleic acid sequence selected from any of SEQ ID NO's: 3 to 14;
e) a nucleic acid sequence sharing at least 80%, more preferably at least 85% and most preferably at least 90%, 95% or even 99% sequence identity with a nucleic acid sequence according to a), b), c) or d).

2. (Regulating and/or encoding) nucleic acid sequence, selected from one of the following sequences:
a) a (regulating and/or encoding) nucleic acid sequence derived from a sequence according to SEQ ID NO: 39, or an allelic variation thereof, and having 10 to 200, 10 to150, 10 to 100, 10 to 50, 10 to 40 or 10 to 30 nucleic acids in length;
b) a (regulating and/or encoding) nucleic acid sequence complementary to a nucleic acid sequence derived from a sequence according to SEQ ID NO: 39, or an allelic variation thereof, and having 10 to 200, 10 to 150, 10 to 100, 10 to 50, 10 to 40 or 10 to 30 nucleic acids in length;
c) a (regulating and/or encoding) nucleic acid sequence hybridizing under stringent conditions to a nucleic acid sequence derived from a sequence according to SEQ ID NO: 39, or an allelic variation thereof, and having 10 to 200, 10 to 150, 10 to 100, 10 to 50, 10 to 40 or 10 to 30 nucleic acids in length;
d) a (regulating and/or encoding) nucleic acid sequence sharing at least 80%, more preferably at least 85% and most preferably at least 90%, 95% or even 99% sequence identity with a nucleic acid sequence according to a), b) or c).

3. Nucleic acid sequence according to any of claims 1 to 3, **characterized in that** the nucleic acid sequence is be labelled for further detection, wherein the label is selected from:
(i) radioactive labels selected from radioactive phosphorylation or a radioactive label selected from radioactive isotopes of sulphur, hydrogen, carbon, nitrogen, iod;
(ii) digoxygenin and digoxygenin-based detection systems;
(iii) fluorescent groups, wherein the fluorescent group isselected from any fluorescent protein, e.g. from a group comprising flùorescein, the blue fluorescent protein (BFP), the green fluorescent protein (GFP), the photo activatable-GFP (PA-GFP), the yellow shifted green fluorescent protein (Yellow GFP), the yellow fluorescent protein (YFP), the enhanced yellow fluorescent protein (EYFP), the cyan fluorescent protein (CFP), the enhanced cyan fluorescent protein (ECFP), the monomeric red fluorescent protein (mRFP1), the kindling fluorescent protein (KFP1), aequorin, the autofluorescent proteins (AFPs), or the fluorescent proteins JRed, TurboGFP, PhiYFP and PhiYFP-m, tHc-Red (HcRed-Tandem), PS-CFP2 and KFP-Red (all available from EVRΩGEN, see also www.evrogen.com), or Alexa 350, Alexa 430, AMCA, BODIPY 630/650, BODIPY 650/665, BODIPY-FL, BODIPY-R6G, BODIPY-TMR, BODIPY-TRX, carboxyfluorescein, Cascade Blue, Cy3, Cy5, 6-FAM, Fluorescein, HEX, 6-JOE, Oregon Green 488, Oregon Green 500, Oregon Green 514, Pacific Blue, REG, Rhodamine Green, Rhodamine Red, ROX, TAMRA, TET, Tetramethylrhodamine, or Texas Red, or other suitable fluorescent proteins;
(iv) chemoluminescent groups including lanthanoid complexes;
(v) metal colloids (e.g. gold, silver, etc) as particles;
(vi) groups for immobilization on a solid phase (e.g. biotin, streptag, antibodies, antigene, etc.); and
(vii) a combination of labels of two or more of the labels mentioned under (i) to (vi).

4. Amino acid sequence encoded by a nucleic acid sequences according to claim 1 or 2.

5. Amino acid sequence selected from:
a) an amino acid sequence according to SEQ ID NO: 2, having 10 to 175, 10 to 150, 10 to 100, 10 to 50, 10 to 25 or 10 to 15 amino acids in length;
b) an amino acid sequence, sharing at least 80%, more preferably at least 85% and most preferably at least 90%, 95% or even 99% sequence identity with an amino acid sequence according to a);
c) an amino acid sequence, sharing up to 80%, more preferably up to 85% and most preferably up to 90%, up to 95% or even up to 99% sequence identity with an amino acid sequence according SEQ ID NO: 2.

6. A vector comprising the nucleic acid sequence according to claim 1 or 2.

7. A vector according to claim 6, wherein the vector is a viral vector suitable for gene-therapy selected from adenoviral vectors, adenovirus-associated viral vectors, alphaviruses such as Semliki Forest Virus and Sindbis Virus, herpes viral vectors, parvovirus vectors, pox virus vectors, pox viruses, such as canary pox virus or vaccinia virus retroviruses, or a viral particle.

8. Antibody, which specifically binds to a CITED4-polypeptide according to SEQ ID NO: 2, or to a nucleic acid sequence according claims 1 or 2 or to an amino acid sequence according to claim 4 or 5.

9. Antibody according to claim 8, wherein the antibody is a polyclonal, monoclonal or anti-idiotype antibody or a fragment of such antibodies.

10. Method for detecting cancer of the central nervous system by detecting the expression of CITED4-nucleic acid sequences in a biological sample, comprising the steps of:
(a) contacting nucleic acid sequences according to claims 1 or 3 as a probe molecule under stringent hybridizing conditions with either
(i) a biological sample, or
(ii) RNA molecules isolated from a biological sample, or
(iii) nucleic acid molecules synthesized from these RNA molecules, wherein these nucleic acid molecules hybridize under stringent conditions with a nucleic acid sequence according to SEQ ID NO: 1, or its complementary strand;
(b) optionally amplifying the hybrids formed according to step a); and
(c) detecting the formation of hybrids of the nucleic acid sequence and the RNA molecules or optionally their amplification products, wherein the presence of the hybrids or optionally their amplification products indicate the expression of CITED4-nucleic acid sequences in the biological sample.

11. Method according to claim 10, wherein the amplification is carried out using PCR, RT-PCR, real-time PCR (RQ-PCR) or primer extension methods.

12. Method for detecting cancer of the central nervous system by detecting the expression of CITED4-nucleic acid sequences in a biological sample, comprising the steps of:
(a) contacting an antibody or antibody fragment according to any of claims 8 or 9 with either
(i) a biological sample,
(ii) RNA molecules isolated from the biological sample,
(iii) nucleic acid molecules synthesized from these RNA molecules, or
(iv) a CITED4-expression product; and
(b) detecting any of the bound antibody or bound antibody fragment.

13. Method for determining the degree of methylation of the complete or partial regulating and/or encoding sequence of CITED4 in a biological sample, comprising the steps of:
(a) optionally isolating the complete or partial regulating and/or encoding sequence of CITED4 from a biological sample;
(b) treating the isolated regulating and/or encoding sequence of CITED4 with a deaminating agent;
(c) contacting the deaminated isolated regulating and/or encoding sequence of CITED4 with a (regulating and/or encoding) nucleic acid sequence according to claims 2 or 3;
(d) amplifying the deaminated isolated regulating and/or encoding sequence of CITED4; and
(e) sequencing the amplified products from step d), and
(f) determining the methylation status by comparing the obtained sequence with the native, non-deaminated sequence of the regulating and/or encoding sequence of CITED4.

14. Method according to claim 13, wherein the demethylating agent is sodium bisulfite (sodium hydrogen sulfite (NAHSO₃)), or 5-aza-2'-deoxycytidine.

15. Method according to any of claims 10 to 14, wherein the biological sample is derived from human or animal origin and includes any body fluids selected from brain fluids, lymph fluids, blood, or urine, tissue samples including brain tissue, tumor tissue, cancers from the central nervous system selected from brain tumors, oligodendroglial tumors, samples derived from bones, connective tissue, bone marrow, and cell lysates derived from any human or animal tissue.

16. Kit comprising at least one container comprising nucleic acid sequences according to claims 1 to 3 and/or an antibody or antibody fragment according to claims 8 or 9.

17. Kit according to claim 16, further comprising a second container comprising one or more indicator reagents capable of indicating the presence of nucleic acid according to claims 1 to 3 and/or a CITED4 antibody or antibody fragments according to claims 8 or 9.

18. Use of nucleic acid sequences according to claims 1 to 3, amino acid sequences according to claims 4 or 5, vectors according to claims 6 or 7, antibodies according to claims 8 or 9, as well as kits according to claims 16 or 17 for the preparation of a pharmaceutical composition for treating tumors of the central nervous system.

19. Use according to claim 18, wherein the tumors of the central nervous system are selected from tumors of the central nervous system, including brain tumors, oligodendroglial tumors, gliomas, oligodendrogliomas, astrocytomas, ependymomas and/or mixed gliomas selected from oligoastrocytomas, and neuroblastomas.

20. Use of nucleic acid sequences according to claims 1 to 3, amino acid sequences according to claims 4 or 5, vectors according to claims 6 or 7, antibodies according to claims 8 or 9, as well as kits according to claims 16 or 17 for gene-therapy.
